# EUROPEAN PATENT APPLICATION

(11) **EP 4 446 344 A1**
(43) Date of publication of application: **16.10.2024**
(21) Application number: 22903488.9
(22) Date of filing: 07.12.2022
(51) Int. Cl.: C07K 16/30, A61K 39/395

(54) **ANTI-CD24 ANTIBODY AND USE THEREOF**

(30) Priority: 07.12.2021 CN 202111487167
(71) Applicant: BEIJING KANGHONG BIOMEDICAL CO., LTD, Beijing 100176 (CN)
(72) Inventor: FENG, Xiao, Chengdu, Sichuan 610036 (CN); KE, Xiao, Chengdu, Sichuan 610036 (CN); LI, Jianhong, Chengdu, Sichuan 610036 (CN); REN, Pengfei, Chengdu, Sichuan 610036 (CN); LEI, Gang, Chengdu, Sichuan 610036 (CN)
(74) Representative: Huang, Liwei
(86) International application number: PCT/CN2022/137123
(87) International publication number: WO 2023/104066

(57) **Abstract**

Provided are an anti-CD24 antibody or an antigen-binding fragment thereof, a pharmaceutical composition and an immunoconjugate containing the antibody or antigen-binding fragment, a nucleic acid molecule encoding the antibody or antigen-binding fragment thereof, a vector containing the nucleic acid molecule, a host cell containing the vector, a method for preparing the antibody or antigen-binding fragment, and the biomedical use of the antibody or antigen-binding fragment, comprising preventing or treating a tumor (such as malignant tumor) or an immune disease.

## Description

### References to relevant applications

The present application claims the priority of the Chinese Patent Application No. 202111487167.1 entitled "An anti-CD24 antibody and use thereof" filed on December 7, 2021, and the patent application mentioned above is incorporated herein by reference in its entirety for all purposes.

### Field of the invention

The present application relates to the field of biotechnology, in particular antibody engineering technology, and specifically relates to anti-CD24 antibodies and use thereof.

### Background of the invention

Under normal circumstances, the immune system can recognize and eliminate tumor cells in the tumor microenvironment. Under the immune selection pressure of the body, tumor cells can adopt different strategies, such as relying on their own high mutation characteristics to evade immune surveillance and gradually establish an immunosuppressive microenvironment to resist and suppress the anti-tumor immune response of the body, so as to break through the limitation and continue to expand. Eventually, they develop into a clinically visible tumor.

Tumor immunotherapy is a treatment method to control and eliminate tumors by restarting and maintaining the tumor-immune cycle and restoring the body's normal anti-tumor immune response. Such tumor immunotherapy includes monoclonal antibody immune checkpoint inhibitors, therapeutic antibodies, cancer vaccines, cell therapy and small molecule inhibitors. It has been widely used in many tumors such as solid tumors of melanoma, non-small cell lung cancer, kidney cancer and prostate cancer. Tumor-targeting antibodies are among the most widely used immunotherapies in the treatment of blood cancers and solid tumors due to their high specificity and excellent pharmacokinetic characteristics. Anti-tumor antibodies can exert anti-tumor properties through several mechanisms, including: (1) enhancing/activating Fc-mediated effectors (ADCC, ADCP, CDC); (2) blocking tumor growth signals; (3) inhibition of tumor angiogenesis; (4) inducing tumor cell apoptosis signal; (5) activating immune cells

CD24, a highly glycosylated protein with an apparent molecular weight between 30 and 70 kDa, is anchored to the membrane by glycosylphosphatidylinositol (GPI). In humans, CD24 is a short peptide containing only 31 amino acids with 16 potential O- and N-glycosylation sites. It is mainly expressed on the cells of hematopoietic lineage, such as B lymphocytes, monocytes, granulocytes and activated T lymphocytes, and can be used as a marker to judge the maturity of a variety of cells (such as T cells, B cells, etc.). It is involved in regulating the body's immune response, and is closely related to autoimmune diseases such as systemic lupus erythematosus and rheumatoid arthritis. In addition, CD24 molecule can be detected at a high level on the cell surface of most malignant tumors such as adenocarcinoma, ovarian cancer, bladder cancer, prostate cancer, non-small cell lung cancer, nasopharyngeal cancer and other tumor tissues. Its expression level is closely related to the occurrence and development of tumors, so that CD24 has been identified as a cancer stem cell marker for many cancer types.

CD24 has been identified as a ligand for P-selectin, which is mainly expressed on the activated vascular endothelial surface and is an important adhesion molecule that promotes cell migration. CD24 on the surface of tumor cells acts as the ligand of P-selectin which mediates the binding of tumor cells to activated platelets and endothelial cells, and promotes the spread, invasion and metastasis of tumor cells. Secondly, as a cell membrane surface signal transduction molecule, CD24 can mediate the proliferation, adhesion, metastasis and invasion of tumor cells through various mechanisms of action. Therefore, CD24 is expected to be a new target for tumor immunotherapy.

At present, there are few reports on the use of anti-CD24 antibody as therapeutic antibody, and there is an urgent need in the art for drug development with CD24 as therapeutic target.

### Summary of the invention

In the first aspect, the present application provides an anti-CD24 antibody or an antigen-binding fragment thereof, wherein the anti-CD24 antibody or antigen-binding fragment thereof comprises a heavy chain variable region and a light chain variable region, wherein the heavy chain variable region comprises heavy chain CDR1 (HCDR1), heavy chain CDR2 (HCDR2) and heavy chain CDR3 (HCDR3), and the light chain variable region comprises light chain CDR1 (LCDR1), light chain CDR2 (LCDR2) and light chain CDR3 (LCDR3), wherein:
the amino acid sequence of HCDR1 comprises X₁GYX₂WH (SEQ ID NO: 152), wherein X₁ is R, N or S, X₂ is S or T; or the amino acid sequence of HCDR1 is TYGVS (SEQ ID NO:5);
the amino acid sequence of HCDR2 comprises X₁X₂X₃X₄X₅GX₆TX₇YX₈X₉X₁₀LX₁₁X₁₂ (SEQ ID NO:153), wherein X₁ is Y, V or F, X₂ is I or T, X₃ is H, W or Q; X₄ is H, G, Y or F; X₅ is R, S or D; X₆ is S, T, N or A; X₇ is N or K; X₈ is N or H; X₉ is P or S; X₁₀ is S or A; X₁₁ is K or I; X₁₂ is S or N;
the amino acid sequence of HCDR3 comprises GX₁X₂X₃X₄X₅DX₆ (SEQ ID NO: 154), wherein X₁ is S or T; X₂ is N, D or T; X₃ is S, R, W, G or T; X₄ is A, S or G; X₅ is F or L; X₆ is Y or H; or the amino acid sequence of HCDR3 is YYGYDFFAY (SEQ ID NO:25) or YYGYDFFPY (SEQ ID NO:26);
the amino acid sequence of LCDR1 comprises X₁SSQSLLYSX₂X₃QKNYX₄X₅ (SEQ ID NO:155), wherein X₁ is K or Q, X₂ is N, S or K, X₃ is N or D, X₄ is L or S; X₅ is A, T or V; or the amino acid sequence of LCDR1 is RASQDISNYLN(SEQ ID NO:35), SASSSISYMH (SEQ ID NO:36), SASSSLSYMH (SEQ ID NO:37), SASSSVSSSYLY (SEQ IDNO:38) or SASSSVSYMH (SEQ ID NO:39);
the amino acid sequence of LCDR2 comprises X₁X₂SX₃X₄X₅S (SEQ ID NO: 156), wherein X₁ is W, L, D, S or Y., X₂ is A or T; X₃ is T, K, N or R; X₄ is R, K or L; X₅ is E, A, Y or H;
the amino acid sequence of LCDR3 comprises X₁QX₂X₃X₄X₅PX₅X₇ (SEQ ID NO: 157), wherein X₁ is Q, H or F; X₂ is Y, G, R or W; X₃ is Y, S, N or F; X₄ is I, G, S, Tor L; X₅ is Y or L; X₆ is L, F, C, P, H or M; X₇ is T or S;
wherein the sequences of HCDR1-3 and LCDR1-3 are defined according to Kabat.

In the second aspect, the present application provides an anti-CD24 antibody or an antigen-binding fragment thereof, wherein the anti-CD24 antibody or antigen-binding fragment thereof comprises a heavy chain variable region, wherein the heavy chain variable region comprises heavy chain CDR1 (HCDR1), heavy chain CDR2 (HCDR2) and heavy chain CDR3 (HCDR3), wherein:
the amino acid sequence of HCDR1 comprises X₁GYX₂WH (SEQ ID NO: 152), wherein X₁ is R, N or S, X₂ is S or T; or the amino acid sequence of HCDR1 is TYGVS (SEQ ID NO:5);
the amino acid sequence of HCDR2 comprises X₁X₂X₃X₄X₅GX₆TX₇YX₈X₉X₁₀LX₁₁X₁₂ (SEQ ID NO:153), wherein X₁ is Y, V or F, X₂ is I or T, X₃ is H, W or Q; X₄ is H, G, Y or F; X₅ is R, S or D; X₆ is S, T, N or A; X₇ is N or K; X₈ is N or H; X₉ is P or S; X₁₀ is S or A; X₁₁ is K or I; X₁₂ is S or N;
the amino acid sequence of HCDR3 comprises GX₁X₂X₃X₄X₅DX₆ (SEQ ID NO: 154), wherein X₁ is S or T; X₂ is N, D or T; X₃ is S, R, W, G or T; X₄ is A, S or G; X₅ is F or L; X₆ is Y or H; or the amino acid sequence of HCDR3 is YYGYDFFAY(SEQ ID NO:25) or YYGYDFFPY(SEQ ID NO:26);
wherein the sequenes of HCDR1-3 are defined according to Kabat.

In the third aspect, the present application provides an isolated nucleic acid molecule (polynucleotide) encoding the anti-CD24 antibody or antigen-binding fragment as described in the first to second aspects.

In the fourth aspect, the present application provides a vector comprising the nucleic acid molecule (polynucleotide) encoding the anti-CD24 antibody or antigen-binding fragment as described in the first to second aspects.

In the fifth aspect, the present application provides an isolated host cell comprising the nucleic acid molecule as described in the third aspect or the vector as described in the fourth aspect.

In the sixth aspect, the present application provides an immunoconjugate comprising the anti-CD24 antibody or antigen-binding fragment thereof as described in the first to second aspects conjugated to a therapeutic agent.

In the seventh aspect, the present application provides a pharmaceutical composition comprising the anti-CD24 antibody or antigen-binding fragment thereof as described in the first to second aspects or the immunoconjugate as described in the sixth aspect, and a pharmaceutically acceptable excipient, diluent or carrier.

In the eighth aspect, the present application provides a use of the anti-CD24 antibody or antigen-binding fragment thereof in the first to second aspects or the nucleic acid molecule in the third aspect or the vector in the fourth aspect or the host cell in the fifth aspect or the immunoconjugate in the sixth aspect in the manufacture of a medicament for the prevention or treatment of a tumor (e.g., malignant tumor) or an immune disease.

In the ninth aspect, the present application provides a method for treating a tumor (e.g., malignant tumor) or an immune disease in an individual, including administering to an individual in need thereof a therapeutic effective amount of the anti-CD24 antibody or antigen-binding fragment thereof as described in the first to second aspects, an immune conjugate as described in the sixth aspect or a pharmaceutical composition as described in the seventh aspect.

In the tenth aspect, the present application provides a method for the preparation of the anti-CD24 antibody or antigen-binding fragment thereof as described in the first to second aspects, including a step for culturing the host cell as described in the fifth aspect.

### Brief description of the drawings

Figure 1A- Figure 1G show the change in tumor volume over time in tumor-bearing mice treated with different exemplary anti-CD24 antibodies of the present application.
Figure 2A- Figure 2F show the tumor weight of tumor-bearing mice at the end of the experiment after treatment with different exemplary anti-CD24 antibodies of the present application.

### Detail description of the invention

### Definition

Unless otherwise defined, all technical terms used herein have the same meaning as understood by those of ordinary skilled in the art. For definitions and terms in this field, those skilled may refer to the Current Protocols in Molecular Biology (Ausubel). The abbreviation for the amino acid residues is a standard 3-letter and/or 1-letter code used in the art to refer to one of the 20 commonly used L-amino acids.

Notwithstanding the numerical ranges and parameter approximations shown in the broad scope of the present application, the numerical values shown in the specific examples are recorded as accurately as possible. However, any numerical value is inherently bound to contain a certain amount of error, which is due to the standard deviation in their respective measurements. In addition, all the scopes disclosed herein should be understood to cover any and all subscopes contained therein. For example, a recorded range of " 1 to 10" shall be considered to contain any and all sub-ranges between the minimum value 1 and the maximum value 10 (including endpoints); i.e., all subranges that start with a minimum value of 1 or greater, such as 1 to 61, and end with a maximum value of 10 or less, such as 5.5 to 10. In addition, any reference referred to as "incorporated herein" shall be understood to be incorporated in its entirety.

Broadly speaking, "antibody" can refer to an immunoglobulin molecule that is able to specifically bind to a target via at least one antigen-recognition site located in the variable region of the immunoglobulin molecule, and thus encompasses a complete antibody, a single chain of an antibody or any antigen-binding fragment of an antibody (also called an "antigen-binding part"). When "antibody" and "antigen-binding fragment/antigen-binding part" appear in the same context, "antibody" can be understood as a complete whole relative to "antigen-binding fragment/antigen-binding part", which together correspond to the broad concept of antibody.

"Full-length antibody" means a protein containing at least two heavy (H) chains and two light (L) chains interconnected by disulfide bonds. Each heavy chain contains a heavy chain variable region (abbreviated VH) and a heavy chain constant region. The heavy chain constant region contains three domains, CH1, CH2, and CH3. Each light chain comprises a light chain variable region (abbreviated VL) and a light chain constant region. The light chain constant region comprises a domain, CL. The VH and VL regions can also be subdivided into regions with high variability, known as complementary determining regions (CDR), interscattered with more conservative regions known as frame regions (FR). Each VH and VL consists of three CDRs and four FRs, arranged from the amino terminal to the carboxyl terminal in the following order: FR1, CDR1, FR2, CDR2, FR3, CDR3, FR4. These variable regions of the heavy and light chains contain binding domains that interact with the antigen. The constant region of antibodies mediates the binding of immunoglobulins to tissues or factors of the host, including various cells of the immune system (such as effector cells) and the first component of the classical complement system (Clq). Full-length antibodies can be any kind of antibody, such as IgD, IgE, IgG, IgA or IgM (or subclasses of the above), but antibodies do not need to belong to any specific class. Depending on the amino acid sequence of the antibody in the constant domain of the heavy chain, immunoglobulins can be assigned to different classes. In general, there are five main classes of immunoglobulins: IgA, IgD, IgE, IgG, and IgM, and several of these classes can be further divided into subclasses (isotypes), such as IgG1, IgG2, IgG3, IgG4, IgA1, and IgA2. The heavy chain constant domains corresponding to different classes of immunoglobulins are respectively called α, δ, ε, γ and µ. The subunit structure and three-dimensional structure of different classes of immunoglobulins are well known. Chimeric or humanized antibodies are also covered in antibodies according to the present application. It is well known to those skilled in the art that the complementary determining region (CDR, generally CDR1, CDR2, and CDR3) is the variable region that has the greatest influence on the affinity and specificity of an antibody. The CDR amino acid sequence of VH or VL is defined in two common ways, namely the Chothia definition and the kabat definition. For the variable region amino acid sequence of a given antibody, it is usually possible to determine the CDR amino acid sequence in the VH and VL amino acid sequences based on the Chothia definition or the Kabat definition. In the examples of the present application, Kabat is used to define the CDR amino acid sequence. For the variable region amino acid sequence of a given antibody, the CDR amino acid sequence of the variable region amino acid sequence can be analyzed in various ways, for example, the online software Abysis can be used to determine (http://www.abysis.org/).

As used herein, the term "antigen-binding fragment" means, in particular, an antibody fragment such as Fv, Fab, F(ab') or Fab, or any fragment that, by chemical modification or incorporation into liposomes, should be capable of increasing half-life, preferably consisting of or comprising a sequence of a part of the variable chain of the heavy or light chain of the antibody from which it is derived. The part of the sequence is sufficient to retain the same binding specificity and sufficient affinity as its source antibody, and such functional fragments will contain a minimum of 5 amino acids, preferably 10, 15, 25, 50, and 100 consecutive amino acids of the antibody sequence from which it is derived. Examples of antigen-binding fragments include, but are not limited to: (1) Fab fragments, which may be univalent fragments with VL-CL chains and VH-CH1 chains; (2) F(ab')₂ segment, which may be a bivalent segment having two Fab' segments connected by a disulfide bridge in the hinge region (i.e., a dimer of Fab'); (3) Fv fragments with the VL and VH domains of a single arm of an antibody.

The term "single chain antibody (scFv)" refers to a single polypeptide chain connected by the VH and VL domains via peptide linkers. (scFv)₂ comprises two VH domains connected by peptide linkers and two VL domains that are combined with the two VH domains via a disulfide bridge.

The terms "Fc fragment", "Fc domain", "Fc portion" or similar terms refer to a portion of the constant region of the antibody heavy chain, including the hinge region, the CH2 fragment and the CH3 fragment of the constant region.

The term "specific binding", as used herein, refers to a non-random binding reaction between two molecules, such as the binding of an antibody to an antigen epitope.

The term "humanized antibody" refers to an antibody obtained by grafting a CDR sequence from another mammalian species, such as a mouse germline, onto a human frame sequence. Other frame area modifications can be made in the human frame sequence. Humanized antibodies or fragments thereof according to the present application may be prepared by means of techniques known to those skilled in the art.

The term "chimeric antibody" refers to an antibody in which the variable region sequence is derived from one species and the constant region sequence is derived from another, such as an antibody in which the variable region sequence is derived from a mouse antibody and the constant region sequence is derived from a human antibody. Chimeric antibodies or fragments thereof according to the present application may be prepared by using gene recombination techniques. For example, the chimeric antibody may be produced by cloning recombinant DNA containing a promoter and a sequence encoding a variable region of a non-human, especially mouse monoclonal antibody according to the present application, and a sequence encoding a constant region of a human antibody. The proposed chimeric antibody encoded by such a recombinant gene would be, for example, a murine-human chimera whose specificity is determined by a variable region derived from mouse DNA and the isotype thereof is determined by a constant region derived from human DNA.

As used herein, the term "monoclonal antibody" refers to an antibody obtained from a population that is essentially homogenous, that is, the individual antibodies that make up the population are identical except for mutations that may occur naturally in a small number of individuals. Monoclonal antibodies referred to herein include, in particular, "chimeric" antibodies in which part of the heavy and/or light chain is identical or homologous to the corresponding amino acid sequence in an antibody derived from a particular species or belonging to a particular antibody class or subclass, and the remainder of the heavy and/or light chain is identical or homologous to the corresponding amino acid sequence in an antibody derived from another species or belonging to another antibody class or subclass. It also includes fragments of such antibodies, provided that they exhibit the desired biological activity.

The term "bispecific antibody" refers to an antibody that has the ability to bind two epitopes simultaneously. The two epitopes can be on different antigens or on the same antigen. Bispecific antibodies can have a variety of structural configurations. For example, a bisspecific antibody may consist of two Fc fragments and two binding parts fused to each of them (similar to a natural antibody, except that the two arms bind to different antigenic targets or epitopes), and the antigen-binding part may be in the form of a single chain antibody (scfv) or Fab fragment.

The term "pharmaceutical composition" as used herein refers to a combination of at least one drug and optionally a pharmaceutically acceptable carrier or excipient combined together to achieve a particular purpose. In some embodiments, said pharmaceutical compositions include combinations that are separated in time and/or space to the extent that they work together to achieve the purpose of the present application. For example, the components contained in the pharmaceutical composition (such as antibodies, nucleic acid molecules, nucleic acid molecular combinations and/or conjugates according to the present application) may be administered to an individual as a whole or separately. When the components contained in the pharmaceutical composition are administered separately to the individual, the components may be administered simultaneously or sequentially to the individual. Preferably, the pharmaceutically acceptable carrier is water, buffer aqueous solution, isotonic salt solution such as starch, magnesium stearate, cellulose, magnesium carbonate, 0.3% glycerol, hyaluronic acid, ethanol or polyalkylene glycol such as polypropylene glycol, triglyceride, etc. The type of pharmaceutically viable carrier used depends in particular on whether the composition according to the present application is formulated for oral, nasal, intradermal, subcutaneous, intramuscular or intravenous administration. Compositions according to the present application may include wetting agents, emulsifiers or buffer substances as additives. Pharmaceutical compositions or pharmaceutical preparations according to the present application may be administered by any suitable route, such as oral, nasal, intradermal, subcutaneous, intramuscular or intravenous administration.

The term "therapeutic effective dose" or "effective dose" as used herein refers to a dose sufficient to demonstrate its benefit to the individual administered. The actual amount of administration, as well as the rate and time course of administration, will depend on the individual's condition and severity. The prescribing of treatments (e.g., decisions on dosage, etc.) is ultimately the responsibility and reliance of the general practitioner and other physicians, often taking into account the disease being treated, the individual patient's condition, the site of delivery, the method of administration and other factors known to the physician.

The term "individual" as used herein refers to mammals, such as humans, but may also refer to other animals, such as wild animals (such as herons, storks, cranes, etc.), domestic animals (such as ducks, geese, etc.) or laboratory animals (such as orangutans, monkeys, rats, mice, rabbits, guinea pigs, groundhogs, ground squirrels, etc.).

The term "homology/identity/consistency" for an amino acid or nucleic acid sequence is defined as the percentage of residues that are identical in an amino acid or nucleotide sequence variant after sequence alignment and introduction of a vacancy, if desired, to achieve the maximum percentage homology. The methods and computer programs used for the alignment are well known in the art.

In general, for the preparation of monoclonal antibodies or their functional fragments, especially murine monoclonal antibodies or their functional fragments, reference may be made to the techniques described especially in the manual "Antibodies" (Harlow and Lane, Antibodies:A Laboratory Manual, Cold Spring Harbor Laboratory, Cold Spring Harbor NY, pp.726,1988) or to the technique described by Kohler and Milstein for the preparation of hybridoma cells (Nature, 256:495-497, 1975).

In the first aspect, the present application provides an anti-CD24 antibody or an antigen-binding fragment thereof, wherein the anti-CD24 antibody or antigen-binding fragment thereof comprises a heavy chain variable region and a light chain variable region, wherein the heavy chain variable region comprises heavy chain CDR1 (HCDR1), heavy chain CDR2 (HCDR2) and heavy chain CDR3 (HCDR3), and the light chain variable region comprises light chain CDR1 (LCDR1), light chain CDR2 (LCDR2) and light chain CDR3 (LCDR3), wherein:
the amino acid sequence of HCDR1 comprises X₁GYX₂WH (SEQ ID NO: 152), wherein X₁ is R, N or S, X₂ is S or T; or the amino acid sequence of HCDR1 is TYGVS (SEQ ID NO:5);
the amino acid sequence of HCDR2 comprises X₁X₂X₃X₄X₅GX₆TX₇YX₈X₉X₁₀LX₁₁X₁₂ (SEQ ID NO:153), wherein X₁ is Y, V or F, X₂ is I or T, X₃ is H, W or Q; X₄ is H, G, Y or F; X₅ is R, S or D; X₆ is S, T, N or A; X₇ is N or K; X₈ is N or H; X₉ is P or S; X₁₀ is S or A; X₁₁ is K or I; X₁₂ is S or N;
the amino acid sequence of HCDR3 comprises GX₁X₂X₃X₄X₅DX₆ (SEQ ID NO: 154), wherein X₁ is S or T; X₂ is N, D or T; X₃ is S, R, W, G or T; X₄ is A, S or G; X₅ is F or L; X₆ is Y or H; or the amino acid sequence of HCDR3 is YYGYDFFAY (SEQ ID NO:25) or YYGYDFFPY (SEQ ID NO:26);
the amino acid sequence of LCDR1 comprises X₁SSQSLLYSX₂X₃QKNYX₄X₅ (SEQ ID NO:155), wherein X₁ is K or Q, X₂ is N, S or K, X₃ is N or D; X₄ is L or S; X₅ is A, T or V; or the amino acid sequence of LCDR1 is RASQDISNYLN (SEQ ID NO:35), SASSSISYMH (SEQ ID NO:36), SASSSLSYMH (SEQ ID NO:37), SASSSVSSSYLY (SEQ IDNO:38) or SASSSVSYMH (SEQ ID NO:39);
the amino acid sequence of LCDR2 comprises X₁X₂SX₃X₄X₅S (SEQ ID NO: 156), wherein X₁ is W, L, D, S or Y; X₂ is A or T; X₃ is T, K, N or R; X₄ is R, K or L; X₅ is E, A, Y or H;
the amino acid sequence of LCDR3 comprises X₁QX₂X₃X₄X₅PX₆X₇ (SEQ ID NO: 157), wherein X₁ is Q, H or F; X₂ is Y, G, R or W; X₃ is Y, S, N or F; X₄ is I, G, S, T or L; X₅ is Y or L; X₆ is L, F, C, P, H or M; X₇ is T or S.

In some embodiments, HCDR1 has any one of the amino acid sequences in SEQ ID NO: 1-5, HCDR2 has any one of the amino acid sequences in SEQ ID NO:6-18, and HCDR3 has any one of the amino acid sequences in SEQ ID NO:19-26; LCDR1 has any one of the amino acid sequences in SEQ ID NO:27-39, LCDR2 has any one of the amino acid sequences in SEQ ID NO:40-47, and LCDR3 has any one of the amino acid sequences in SEQ ID NO:48-56.

In some embodiments, the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 35, SEQ ID NO:47, SEQ ID NO: 53, respectively;
the amino acid sequences of HCDR1-HCDR3 are SEQ ID NO: 1, SEQ ID NO: 17, SEQ ID NO: 21, respectively, and the amino acid sequences of LCDR1-LCDR3 are SEQ ID NO: 27, SEQ ID NO: 43, SEQ ID NO: 55, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 28, SEQ ID NO: 45, SEQ ID NO: 48, respectively;
the amino acid sequences of HCDR1-HCDR3 are SEQ ID NO: 1, SEQ ID NO: 6, SEQ ID NO: 19, respectively, and LCDR1-LCDR3 are SEQ ID NO: 28, SEQ ID NO: 41, SEQ ID NO: 48, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 13, SEQ ID NO: 20, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 16, SEQ ID NO: 24, respectively, and the amino acid sequences of LCDR1-LCDR3 are SEQ ID NO: 34, SEQ ID NO: 40, SEQ ID NO: 49, respectively;
the amino acid sequences of HCDR1-HCDR3 are SEQ ID NO: 5, SEQ ID NO: 9, SEQ ID NO: 25, respectively, and the amino acid sequences of LCDR1-LCDR3 are SEQ ID NO: 37, SEQ ID NO: 42, SEQ ID NO: 50, respectively;
the amino acid sequences of HCDR1-HCDR3 are SEQ ID NO: 5, SEQ ID NO: 9, SEQ ID NO: 26, respectively, and the amino acid sequences of LCDR1-LCDR3 are SEQ ID NO: 39, SEQ ID NO: 42, SEQ ID NO: 50, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 32, SEQ ID NO: 40, SEQ ID NO: 48, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 1, SEQ ID NO: 10, SEQ ID NO: 21, respectively, and the amino acid sequences of LCDR1-LCDR3 are SEQ ID NO: 31, SEQ ID NO: 46, SEQ ID NO: 49, respectively;
the amino acid sequences of HCDR1-HCDR3 are SEQ ID NO: 1, SEQ ID NO: 10, SEQ ID NO: 21, respectively, and the amino acid sequences of LCDR1-LCDR3 are SEQ ID NO: 27, SEQ ID NO: 43, SEQ ID NO: 56, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 30, SEQ ID NO: 40, SEQ ID NO: 49, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 30, SEQ ID NO: 40, SEQ ID NO: 49, respectively;
the amino acid sequences of HCDR1-HCDR3 are SEQ ID NO: 3, SEQ ID NO: 12, SEQ ID NO: 22, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 3, SEQ ID NO: 18, SEQ ID NO: 22, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 31, SEQ ID NO: 40, SEQ ID NO: 54, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 3, SEQ ID NO: 15, SEQ ID NO: 20, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 3, SEQ ID NO: 15, SEQ ID NO: 20, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 33, SEQ ID NO: 40, SEQ ID NO: 49, respectively;
the amino acid sequences of HCDR1-HCDR3 are SEQ ID NO: 4, SEQ ID NO: 11, SEQ ID NO: 23, respectively, and the amino acid sequences of LCDR1-LCDR3 are SEQ ID NO: 33, SEQ ID NO: 40, SEQ ID NO: 49, respectively;
the amino acid sequences of HCDR1-HCDR3 are SEQ ID NO: 4, SEQ ID NO: 11, SEQ ID NO: 23, respectively, and the amino acid sequences of LCDR1-LCDR3 are SEQ ID NO: 32, SEQ ID NO: 40, SEQ ID NO: 48, respectively;
the amino acid sequences of HCDR1-HCDR3 are SEQ ID NO: 1, SEQ ID NO: 14, SEQ ID NO: 19, respectively, and the amino acid sequences of LCDR1-LCDR3 are SEQ ID NO: 29, SEQ ID NO: 40, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 29, SEQ ID NO: 40, SEQ ID NO: 48, respectively;
the amino acid sequences of HCDR1-HCDR3 are SEQ ID NO: 3, SEQ ID NO: 12, SEQ ID NO: 22, respectively, and the amino acid sequences of LCDR1-LCDR3 are SEQ ID NO: 29, SEQ ID NO: 40, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 38, SEQ ID NO: 44, SEQ ID NO: 52, respectively; or
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 36, SEQ ID NO: 42, SEQ ID NO: 51, respectively.

In some embodiments, the heavy chain variable region has the amino acid sequence as set forth in any one of SEQ ID NO: 57-71 and 98-120 or an amino acid sequences with at least 80%, 81%, 82%, 83%, 84%, 85%, 86%, 87%, 88%, 90%, 91%, 92%, 93%, 94%, 95%, 96%, 97%, 98% or 99% homology to the amino acid sequence as set forth in any one of SEQ ID NO: 57-71 and 98-120.

In some embodiments, the amino acid sequence in the heavy chain variable region differs from the amino acid sequence as set forth in any one of SEQ ID NO: 57-71 and 98-120 by about 1, 2, 3, 4, 5, 6, 7, 8, 9 or 10 amino acids in substitution, deletion and/or addition. In some embodiments, the C-terminal or N-terminal region of the amino acid sequence as set forth in any one of 57-71 and 98-120 can be truncated by approximately 1, 2, 3, 4, 5, 6, 7, 8, 9, 10, 11, 12, 13, 14, 15, 20, 25 or more amino acids while still maintaining similar function as the heavy chain variable region of the antibody. In some embodiments, 1, 2, 3, 4, 5, 6, 7, 8, 9, 10, 11, 12, 13, 14, 15, 20, 25 or more amino acids can be added to the C-terminal or N-terminal region of the amino acid sequence as set forth in any one of 57-71 and 98-120, the resulting amino acid sequence still maintains similar function as the heavy chain variable region of the antibody. In some embodiments, 1, 2, 3, 4, 5, 6, 7, 8, 9, 10, 11, 12, 13, 14, 15, 20, 25 or more amino acids can also be added or deleted in a region outside the C-terminus or N-terminus of any of the amino acid sequences shown in 57-71 or 98-120, as long as the altered amino acid sequence essentially maintains a similar function as the heavy chain variable region of the antibody.

In some embodiments, the light chain variable region has the amino acid sequence as set forth in any one of SEQ ID NO: 72-94 and 121-147 or an amino acid sequences with at least 80%, 81%, 82%, 83%, 84%, 85%, 86%, 87%, 88%, 90%, 91%, 92%, 93%, 94%, 95%, 96%, 97%, 98% or 99% homology to the amino acid sequence as set forth in any one of SEQ ID NO: 72-94 and 121-147.

In some embodiments, the amino acid sequence in the variable region of the light chain differs from the amino acid sequence as set forth in any one of SEQ ID NO: 72-94 and 121-147 by about 1, 2, 3, 4, 5, 6, 7, 8, 9 or 10 amino acids in substitution, deletion, and/or addition. In some embodiments, the C-terminal or N-terminal region of the amino acid sequence as set forth in any one of 72-94 and 121-147 can also be truncated by approximately 1, 2, 3, 4, 5, 6, 7, 8, 9, 10, 11, 12, 13, 14, 15, 20, 25 or more amino acids while still maintaining a similar function as the light chain variable region of the antibody. In some embodiments, 1, 2, 3, 4, 5, 6, 7, 8, 9, 10, 11, 12, 13, 14, 15, 20, 25 or more amino acids can be added to the C-terminal or N-terminal region of the amino acid sequence as set forth in any one of 72-94 and 121-147, the resulting amino acid sequence still maintains similar function as the light chain variable region of the antibody. In some embodiments, 1, 2, 3, 4, 5, 6, 7, 8, 9, 10, 11, 12, 13, 14, 15, 20, 25 or more amino acids can also be added or deleted in a region outside the C-terminus or N-terminus of any of the amino acid sequences shown in SEQ ID NO: 72-94 and 121-147, as long as the altered amino acid sequence essentially maintains a similar function as the light chain variable region of the antibody.

In some embodiments, the amino acid sequence of the heavy chain variable region is SEQ ID NO: 57, and the amino acid sequence of the light chain variable region is SEQ ID NO: 72;
the amino acid sequence of the heavy chain variable region is SEQ ID NO: 58, and the amino acid sequence of the light chain variable region is SEQ ID NO: 73;
the amino acid sequence of the heavy chain variable region is SEQ ID NO: 59, and the amino acid sequence of the light chain variable region is SEQ ID NO: 74;
the amino acid sequence of the heavy chain variable region is SEQ ID NO: 60, and the amino acid sequence of the light chain variable region is SEQ ID NO: 75;
the amino acid sequence of the heavy chain variable region is SEQ ID NO: 61, and the amino acid sequence of the light chain variable region is SEQ ID NO: 76;
the amino acid sequence of the heavy chain variable region is SEQ ID NO: 62, and the amino acid sequence of the light chain variable region is SEQ ID NO: 76;
the amino acid sequence of the heavy chain variable region is SEQ ID NO: 63, and the amino acid sequence of the light chain variable region is SEQ ID NO: 77;
the amino acid sequence of the heavy chain variable region is SEQ ID NO: 64, and the amino acid sequence of the light chain variable region is SEQ ID NO: 78;
the amino acid sequence of the heavy chain variable region is SEQ ID NO: 65, and the amino acid sequence of the light chain variable region is SEQ ID NO: 79;
the amino acid sequence of the heavy chain variable region is SEQ ID NO: 59, and the amino acid sequence of the light chain variable region is SEQ ID NO: 80;
the amino acid sequence of the heavy chain variable region is SEQ ID NO: 59, and the amino acid sequence of the light chain variable region is SEQ ID NO: 81;
the amino acid sequence of the heavy chain variable region is SEQ ID NO: 66, and the amino acid sequence of the light chain variable region is SEQ ID NO: 82;
the amino acid sequence of the heavy chain variable region is SEQ ID NO: 66, and the amino acid sequence of the light chain variable region is SEQ ID NO: 83;
the amino acid sequence of the heavy chain variable region is SEQ ID NO: 61, and the amino acid sequence of the light chain variable region is SEQ ID NO: 84;
the amino acid sequence of the heavy chain variable region is SEQ ID NO: 61, and the amino acid sequence of the light chain variable region is SEQ ID NO: 85;
the amino acid sequence of the heavy chain variable region is SEQ ID NO: 67, and the amino acid sequence of the light chain variable region is SEQ ID NO: 86;
the amino acid sequence of the heavy chain variable region is SEQ ID NO: 68, and the amino acid sequence of the light chain variable region is SEQ ID NO: 87;
the amino acid sequence of the heavy chain variable region is SEQ ID NO: 57, and the amino acid sequence of the light chain variable region is SEQ ID NO: 88;
the amino acid sequence of the heavy chain variable region is SEQ ID NO: 61, and the amino acid sequence of the light chain variable region is SEQ ID NO: 89;
the amino acid sequence of the heavy chain variable region is SEQ ID NO: 61, and the amino acid sequence of the light chain variable region is SEQ ID NO: 76;
the amino acid sequence of the heavy chain variable region is SEQ ID NO: 57, and the amino acid sequence of the light chain variable region is SEQ ID NO: 90;
the amino acid sequence of the heavy chain variable region is SEQ ID NO: 69, and the amino acid sequence of the light chain variable region is SEQ ID NO: 76;
the amino acid sequence of the heavy chain variable region is SEQ ID NO: 57, and the amino acid sequence of the light chain variable region is SEQ ID NO: 76;
the amino acid sequence of the heavy chain variable region is SEQ ID NO: 69, and the amino acid sequence of the light chain variable region is SEQ ID NO: 90;
the amino acid sequence of the heavy chain variable region is SEQ ID NO: 59, and the amino acid sequence of the light chain variable region is SEQ ID NO: 76;
the amino acid sequence of the heavy chain variable region is SEQ ID NO: 59, and the amino acid sequence of the light chain variable region is SEQ ID NO: 91;
the amino acid sequence of the heavy chain variable region is SEQ ID NO: 70, and the amino acid sequence of the light chain variable region is SEQ ID NO: 91;
the amino acid sequence of the heavy chain variable region is SEQ ID NO: 70, and the amino acid sequence of the light chain variable region is SEQ ID NO: 80;
the amino acid sequence of the heavy chain variable region is SEQ ID NO: 71, and the amino acid sequence of the light chain variable region is SEQ ID NO: 92;
the amino acid sequence of the heavy chain variable region is SEQ ID NO: 57, and the amino acid sequence of the light chain variable region is SEQ ID NO: 92;
the amino acid sequence of the heavy chain variable region is SEQ ID NO: 67, and the amino acid sequence of the light chain variable region is SEQ ID NO: 92;
the amino acid sequence of the heavy chain variable region is SEQ ID NO: 57, and the amino acid sequence of the light chain variable region is SEQ ID NO: 93;
the amino acid sequence of the heavy chain variable region is SEQ ID NO: 57, and the amino acid sequence of the light chain variable region is SEQ ID NO: 94;
the amino acid sequence of the heavy chain variable region is SEQ ID NO: 101, and the amino acid sequence of the light chain variable region is SEQ ID NO: 137;
the amino acid sequence of the heavy chain variable region is SEQ ID NO: 98, and the amino acid sequence of the light chain variable region is SEQ ID NO: 144;
the amino acid sequence of the heavy chain variable region is SEQ ID NO: 99, and the amino acid sequence of the light chain variable region is SEQ ID NO: 122;
the amino acid sequence of the heavy chain variable region is SEQ ID NO: 114, and the amino acid sequence of the light chain variable region is SEQ ID NO: 143;
the amino acid sequence of the heavy chain variable region is SEQ ID NO: 98, and the amino acid sequence of the light chain variable region is SEQ ID NO: 142;
the amino acid sequence of the heavy chain variable region is SEQ ID NO: 101, and the amino acid sequence of the light chain variable region is SEQ ID NO: 124;
the amino acid sequence of the heavy chain variable region is SEQ ID NO: 98, and the amino acid sequence of the light chain variable region is SEQ ID NO: 122;
the amino acid sequence of the heavy chain variable region is SEQ ID NO: 99, and the amino acid sequence of the light chain variable region is SEQ ID NO: 121;
the amino acid sequence of the heavy chain variable region is SEQ ID NO: 98, and the amino acid sequence of the light chain variable region is SEQ ID NO: 124;
the amino acid sequence of the heavy chain variable region is SEQ ID NO: 98, and the amino acid sequence of the light chain variable region is SEQ ID NO: 137;
the amino acid sequence of the heavy chain variable region is SEQ ID NO: 98, and the amino acid sequence of the light chain variable region is SEQ ID NO: 121;
the amino acid sequence of the heavy chain variable region is SEQ ID NO: 101, and the amino acid sequence of the light chain variable region is SEQ ID NO: 121;
the amino acid sequence of the heavy chain variable region is SEQ ID NO: 101, and the amino acid sequence of the light chain variable region is SEQ ID NO: 133;
the amino acid sequence of the heavy chain variable region is SEQ ID NO: 99, and the amino acid sequence of the light chain variable region is SEQ ID NO: 124;
the amino acid sequence of the heavy chain variable region is SEQ ID NO: 99, and the amino acid sequence of the light chain variable region is SEQ ID NO: 134;
the amino acid sequence of the heavy chain variable region is SEQ ID NO: 111, and the amino acid sequence of the light chain variable region is SEQ ID NO: 136;
the amino acid sequence of the heavy chain variable region is SEQ ID NO: 98, and the amino acid sequence of the light chain variable region is SEQ ID NO: 134;
the amino acid sequence of the heavy chain variable region is SEQ ID NO: 98, and the amino acid sequence of the light chain variable region is SEQ ID NO: 133;
the amino acid sequence of the heavy chain variable region is SEQ ID NO: 99, and the amino acid sequence of the light chain variable region is SEQ ID NO: 133;
the amino acid sequence of the heavy chain variable region is SEQ ID NO: 101, and the amino acid sequence of the light chain variable region is SEQ ID NO: 122;
the amino acid sequence of the heavy chain variable region is SEQ ID NO: 118, and the amino acid sequence of the light chain variable region is SEQ ID NO: 146;
the amino acid sequence of the heavy chain variable region is SEQ ID NO: 99, and the amino acid sequence of the light chain variable region is SEQ ID NO: 137;
the amino acid sequence of the heavy chain variable region is SEQ ID NO: 115, and the amino acid sequence of the light chain variable region is SEQ ID NO: 122;
the amino acid sequence of the heavy chain variable region is SEQ ID NO: 117, and the amino acid sequence of the light chain variable region is SEQ ID NO: 121;
the amino acid sequence of the heavy chain variable region is SEQ ID NO: 116, and the amino acid sequence of the light chain variable region is SEQ ID NO: 145;
the amino acid sequence of the heavy chain variable region is SEQ ID NO: 104, and the amino acid sequence of the light chain variable region is SEQ ID NO: 125;
the amino acid sequence of the heavy chain variable region is SEQ ID NO: 105, and the amino acid sequence of the light chain variable region is SEQ ID NO: 123;
the amino acid sequence of the heavy chain variable region is SEQ ID NO: 110, and the amino acid sequence of the light chain variable region is SEQ ID NO: 126;
the amino acid sequence of the heavy chain variable region is SEQ ID NO: 109, and the amino acid sequence of the light chain variable region is SEQ ID NO: 123;
the amino acid sequence of the heavy chain variable region is SEQ ID NO: 110, and the amino acid sequence of the light chain variable region is SEQ ID NO: 123;
the amino acid sequence of the heavy chain variable region is SEQ ID NO: 104, and the amino acid sequence of the light chain variable region is SEQ ID NO: 130;
the amino acid sequence of the heavy chain variable region is SEQ ID NO: 109, and the amino acid sequence of the light chain variable region is SEQ ID NO: 126;
the amino acid sequence of the heavy chain variable region is SEQ ID NO: 108, and the amino acid sequence of the light chain variable region is SEQ ID NO: 130;
the amino acid sequence of the heavy chain variable region is SEQ ID NO: 105, and the amino acid sequence of the light chain variable region is SEQ ID NO: 126;
the amino acid sequence of the heavy chain variable region is SEQ ID NO: 113, and the amino acid sequence of the light chain variable region is SEQ ID NO: 141;
the amino acid sequence of the heavy chain variable region is SEQ ID NO: 108, and the amino acid sequence of the light chain variable region is SEQ ID NO: 125;
the amino acid sequence of the heavy chain variable region is SEQ ID NO: 103, and the amino acid sequence of the light chain variable region is SEQ ID NO: 139;
the amino acid sequence of the heavy chain variable region is SEQ ID NO: 107, and the amino acid sequence of the light chain variable region is SEQ ID NO: 140;
the amino acid sequence of the heavy chain variable region is SEQ ID NO: 106, and the amino acid sequence of the light chain variable region is SEQ ID NO: 140;
the amino acid sequence of the heavy chain variable region is SEQ ID NO: 106, and the amino acid sequence of the light chain variable region is SEQ ID NO: 129;
the amino acid sequence of the heavy chain variable region is SEQ ID NO: 107, and the amino acid sequence of the light chain variable region is SEQ ID NO: 129;
the amino acid sequence of the heavy chain variable region is SEQ ID NO: 107, and the amino acid sequence of the light chain variable region is SEQ ID NO: 128;
the amino acid sequence of the heavy chain variable region is SEQ ID NO: 103, and the amino acid sequence of the light chain variable region is SEQ ID NO: 128;
the amino acid sequence of the heavy chain variable region is SEQ ID NO: 103, and the amino acid sequence of the light chain variable region is SEQ ID NO: 140;
the amino acid sequence of the heavy chain variable region is SEQ ID NO: 103, and the amino acid sequence of the light chain variable region is SEQ ID NO: 129;
the amino acid sequence of the heavy chain variable region is SEQ ID NO: 120, and the amino acid sequence of the light chain variable region is SEQ ID NO: 139;
the amino acid sequence of the heavy chain variable region is SEQ ID NO: 106, and the amino acid sequence of the light chain variable region is SEQ ID NO: 128;
the amino acid sequence of the heavy chain variable region is SEQ ID NO: 112, and the amino acid sequence of the light chain variable region is SEQ ID NO: 147;
the amino acid sequence of the heavy chain variable region is SEQ ID NO: 112, and the amino acid sequence of the light chain variable region is SEQ ID NO: 138;
the amino acid sequence of the heavy chain variable region is SEQ ID NO: 119, and the amino acid sequence of the light chain variable region is SEQ ID NO: 138;
the amino acid sequence of the heavy chain variable region is SEQ ID NO: 100, and the amino acid sequence of the light chain variable region is SEQ ID NO: 127;
the amino acid sequence of the heavy chain variable region is SEQ ID NO: 102, and the amino acid sequence of the light chain variable region is SEQ ID NO: 127;
the amino acid sequence of the heavy chain variable region is SEQ ID NO: 100, and the amino acid sequence of the light chain variable region is SEQ ID NO: 135;
the amino acid sequence of the heavy chain variable region is SEQ ID NO: 102, and the amino acid sequence of the light chain variable region is SEQ ID NO: 131;
the amino acid sequence of the heavy chain variable region is SEQ ID NO: 100, and the amino acid sequence of the light chain variable region is SEQ ID NO: 132;
the amino acid sequence of the heavy chain variable region is SEQ ID NO: 102, and the amino acid sequence of the light chain variable region is SEQ ID NO: 135;
the amino acid sequence of the heavy chain variable region is SEQ ID NO: 102, and the amino acid sequence of the light chain variable region is SEQ ID NO: 132; or
the amino acid sequence of the heavy chain variable region is SEQ ID NO: 100, and the amino acid sequence of the light chain variable region is SEQ ID NO: 131.

In the second aspect, the present application provides an anti-CD24 antibody or an antigen-binding fragment thereof, wherein the anti-CD24 antibody or antigen-binding fragment thereof comprises a heavy chain variable region, wherein the heavy chain variable region comprises heavy chain CDR1 (HCDR1), heavy chain CDR2 (HCDR2) and heavy chain CDR3 (HCDR3), wherein:
the amino acid sequence of HCDR1 comprises X₁GYX₂WH (SEQ ID NO: 152), wherein X₁ is R, N or S, X₂ is S or T; or the amino acid sequence of HCDR1 is TYGVS (SEQ ID NO:5);
the amino acid sequence of HCDR2 comprises X₁X₂X₃X₄X₅GX₆TX₇YX₈X₉X₁₀LX₁₁X₁₂ (SEQ ID NO:153), wherein X₁ is Y, V or F, X₂ is I or T, X₃ is H, W or Q; X₄ is H, G, Y or F; X₅ is R, S or D; X₆ is S, T, N or A; X₇ is N or K; X₈ is N or H; X₉ is P or S; X₁₀ is S or A; X₁₁ is K or I; X₁₂ is S or N;
the amino acid sequence of HCDR3 consists of GX₁X₂X₃X₄X₅DX₆ (SEQ ID NO: 154), wherein X₁ is S or T; X₂ is N, D or T; X₃ is S, R, W, G or T; X₄ is A, S or G; X₅ is F or L; X₆ is Y or H; or the amino acid sequence of HCDR3 is YYGYDFFAY (SEQ ID NO:25) or YYGYDFFPY (SEQ ID NO:26).

An example of an antibody as described in the second aspect is a single-domain antibody, also known as a Variable domain of heavy chain of heavy-chain antibody (VHH), which consists of only one variable domain of heavy chain, also known as a nanoantibody. VHH antibody is a natural light-chain deficient antibody originally found in the peripheral blood of alpacas, which contains only one heavy-chain variable region (VHH) and two conventional CH2 and CH3 regions. The cloned and expressed VHH structure has the same structural stability and antigen binding activity as the original heavy chain antibody, and is the smallest unit that can bind a target antigen.

In the absence of contradiction, the technical solutions and technical features as described in the first aspect also apply to the second aspect.

In some embodiments of the first to second aspects, the anti-CD24 antibody is a full antibody, a single-chain antibody (scFv) or a bispecific antibody. In some embodiments of the first to second aspects, the antigen-binding fragment of an anti-CD24 antibody is Fab, Fab', Fv or F(ab')₂. In some embodiments of the first to second aspects, the anti-CD24 antibody is a murine antibody, a chimeric antibody, a humanized antibody or a fully human antibody. In some embodiments of the first to second aspects, the anti-CD24 antibody is a monoclonal antibody. In some embodiments of the first to second aspects, the anti-CD24 antibody is a bispecific antibody.

In some embodiments of the first to second aspects, the light chain constant region of an anti-CD24 antibody or antigen-binding fragment thereof is the light chain constant region of a human antibody. In some specific embodiments, the light chain constant region of an anti-CD24 antibody or antigen-binding fragment thereof is selected from human κ type or λ type light chain constant region. In some specific embodiments, the light chain constant region of the anti-CD24 antibody or antigen-binding fragment thereof is human κ type light chain constant region. In some specific embodiments, the human κ type light chain constant region has the amino acid sequence as set forth in SEQ ID NO: 151 or a mutant thereof.

In some embodiments of the first to second aspects, the heavy chain constant region of an anti-CD24 antibody or antigen-binding fragment thereof is the heavy chain constant region of a human antibody. In some embodiments of the first to second aspects, the heavy chain constant region of an anti-CD24 antibody or antigen-binding fragment thereof is selected from any one of the human IgD, IgE, IgM, IgA, IgG1, IgG2a, IgG2b, IgG3, and IgG4 types. In some embodiments of the first to second aspects, the heavy chain constant region of an antibody or antigen-binding fragment thereof is selected from IgG1, IgG2 or IgG4 type. In some specific embodiments, the IgG1 type heavy chain constant region has the amino acid sequence as set forth in SEQ ID NO: 148 or a mutant thereof; the IgG2 heavy chain constant region has the amino acid sequence as set forth in SEQ ID NO: 149 or a mutant thereof; the IgG4 type heavy chain constant region has an amino acid sequence as set forth in SEQ ID NO: 150 or a mutant thereof.

In the third aspect, the present application provides an isolated nucleic acid molecule (polynucleotide) encoding the anti-CD24 antibody or antigen-binding fragment as described in the first to second aspects. In some embodiments, the polynucleotide is operably attached to a regulatory sequence that can be recognized by a host cell transformed with the vector.

In the fourth aspect, the present application provides a vector (e.g., expression vector) comprising the polynucleotide encoding the anti-CD24 antibody or antigen-binding fragment as described in the first to second aspects. The choice of expression vector depends on the selection of the host cell and can be selected to have the desired expression and regulatory characteristics in the selected host cell.

An "expression vector" is a vector that comprises one or more expression control sequences, and an "expression control sequence" is a DNA sequence that controls and regulates transcription and/or translation of another DNA sequence.

Nucleic acids in the vector may be operationally linked to one or more expression control sequences. As used herein, "operationally linked" means incorporated into a genetic construct such that the expression control sequence effectively controls the expression of the target coding sequence. Examples of expression control sequences include promoters, enhancers, and transcription termination regions. A promoter is an expression control sequence consisting of a region of a DNA molecule that is usually within 100 nucleotides upstream of the transcription start point (usually near the start site of RNA polymerase II). In order to keep the coding sequence under the control of the promoter, the translation start site of the peptide translation reading frame must be located between 1 and about 50 nucleotides downstream of the promoter. Enhancers provide expression specificity in terms of time, location, and level. Unlike promoters, enhancers can function when located at different distances from transcription sites. Enhancers may also be located downstream of the transcription start site. When the RNA polymerase is able to transcribe the coding sequence into mRNA, which can then be translated into a protein encoded by the coding sequence, the coding sequence is "operationally connected" to and "under the control" of the expression control sequence in the cell.

Suitable expression vectors include, but are not limited to, plasmids and viral vectors derived from, for example, bacteriophages, baculoviruses, tobacco mosaic viruses, herpes viruses, cytomegaloviruses, retroviruses, vaccinia viruses, adenoviruses, and adeno-associated viruses. Many vectors and expression systems are available from sources such as Novagen (Madison,WI), Clontech (Palo Alto,CA), Stratagene (LaJolla,CA), and Invitrogen LifeTechnologies (Carlsbad,CA), etc.

The expression vector may include a tag sequence. The tag sequence is usually expressed as a fusion with the encoded polypeptide. Such labels can be inserted anywhere within the polypeptide, including carboxyl or amino terminals. Examples of useful labels include, but are not limited to, Fc fragments, polyhistidine, green fluorescent protein (GFP), glutathione S-transferase (GST), c-myc, hemagglutinin, FlagTM labels (Kodak,NewHaven,CT), maltose E-binding proteins, and protein A. In some embodiments, the nucleic acid molecule encoding Siglec-15 fusion peptide is present in a vector containing nucleic acids encoding one or more domains of the Ig heavy chain constant region, such domains are, for example, the amino acid sequence (Fc fragment) corresponding to the hinge region, CH2 region and CH3 region of the human immunoglobulin C γ 1 chain.

In the fifth aspect, the present application provides an isolated host cell comprising the nucleic acid molecule as described in the third aspect or the vector as described in the fourth aspect.

In some embodiments, the host cell may be a prokaryotic host cell, a eukaryotic host cell or a phage. The prokaryotic host cells can be Escherichia coli, Bacillus subtilis, Streptomyces or Proteus mirabilis. The eukaryotic host cells can be mammalian cells, such as 293 cells, CHO cells, BHK cells, COS cells, myeloma cells, etc.; yeast cells, such as Pichia pastoris, Saccharomyces cerevisiae, schizomyces, etc.; insect cells, such as grass armyworms; plant cells, such as tobacco.

In the sixth aspect, the present application provides an immunoconjugate (also referred to as antibody-drug conjugate (ADC)) that contain the anti-CD24 antibody or antigen-binding fragment thereof as described in the first to second aspects conjugated to a therapeutic agent.

In some embodiments, the therapeutic agent is an anti-tumor drug, for example, a cytotoxic drug, an immunoenhancer or a radioisotope.

In some embodiments, the type of the cytotoxic drug includes tubulin inhibitors (such as alkaloids), DNA topoisomerase inhibitors, DNA damaging agents, anti-metabolic drugs or anti-tumor antibiotics.

In some embodiments, the tubulin inhibitors include, but are not limited to, Oristatin derivatives (e.g. MMAE (Monomethyl auristatin E), MMAF (Monomethyl auristatin)F)) or derivatives of mydenin alkaloids (e.g. DM1, DM4, Ansamitocin, Mertansine or dolastatin and derivatives thereof).

In some embodiments, DNA topoisomerase inhibitors are camptothecin analogues or DNA topoisomerase I inhibitors and derivatives thereof, for example, DXD, SN38, Irinotecan, Irinotecan hydrochloride, camptothecin, 9-amino-camptothecin, 9-nitrocamptothecin, 10-hydroxy-camptothecin, 9-chloro-10-hydroxy-camptothecin, 22-hydroxy-camptothecin, Topotecan, Letotecan, Belotecan, Exatecan, homosilatecan, 6,8-dibromo-2-methyl-3-[2-(D-xylopyranylamino)phenyl]-4(3H)-quinazolinone, 2-cyano-3-(3,4-dihydroxyphenyl)-N-(phenylmethyl)-(2E)-2-acrylamide, 2-cyano-3-(3,4-dihydroxyphenyl)-N-(3-hydroxyphenylpropyl)-(E)-2-acrylamide, 12- β -D-glucopyranyl-12,13-dihydro-2,10-dihydroxy-6-[[2-hydroxy-1-(hydroxymethyl)ethyl]amino]-5H-indolo[2,3-a]py rrolo[3,4-c]carbazole-5,7(6H)-diketone, N-[2-(dimethylamine)ethyl]-4-acridine formamide dihydrochloride, N-[2-(dimethylamino)ethyl]-4-acridine formamide.

In some embodiments, DNA damaging agents include, but are not limited to, calicheamicins, duocarmycins, anthracycline derivative PBD (pyrrolobenzodiazepine).

In some embodiments, antimetabolic drugs include, but are not limited to, methotrexate, 6-mercaptopurine or 5-fluorouracil.

In some embodiments, anti-tumor antibiotics include, but are not limited to, polypeptide antibiotics (such as actinomycin D or bleomycin) or anthraquinones (such as doxorubicin or mitoxantrone hydrochloride). In some embodiments, radioisotopes include, but are not limited to, 211At, 131I, 125I, 90Y, 186Re, 188Re, 153Sm, 212Bi, 32P, 60Co or 177Lu.

In some embodiments, immune enhancers include, but are not limited to, levamisole, pidotimod, imiquimod, isopropyl inosine, polyinosinic-polycytidylic acid or polyinosinic-polyuridylic acid.

In some embodiments, the anti-CD24 antibody of the present application is covalently connected to the drug portion by a linker. In some embodiments, the linker is a cleavable linker. In some embodiments, the linker can be cleaved under intracellular conditions. In one embodiment, the linker is hydrolyzed at pH less than 5.5. In some embodiments, the linker may be cut by intracellular proteases. In some embodiments, the linker is a cathepsin cleavable linker. In some embodiments, the linker contains a dipeptide. In some embodiments, the dipeptide is valine (Val)-citrulline (Cit). In some embodiments, the antibody is attached to the linker by the cysteine sulfhydryl group of the antibody.

In the seventh aspect, the present application provides a pharmaceutical composition comprising the anti-CD24 antibody or antigen-binding fragment thereof as described in the first to second aspects or the immunoconjugate as described in the sixth aspect, and a pharmaceutically acceptable excipient, diluent or carrier. In some embodiments, the pharmaceutical composition is an intravitreal injection, a subretinal injection, an intrachoroidal injection, an intravenous injection, an intratumoral injection or an intramuscular injection. In some embodiments, the pharmaceutical composition may also comprise one or more of the following: lubricants, such as talc powder, magnesium stearate and mineral oil; wetting agent; emulsifier; suspending agent; preservatives such as benzoic acid, sorbic acid and calcium propionate; sweeteners and/or flavorings, etc. In some embodiments, the pharmaceutical compositions in the present application can be formulated in the form of tablets, pills, powders, lozenges, elixirs, suspensions, emulsions, solutions, syrups, suppositories or capsules. In some embodiments, the pharmaceutical composition of the present application may be delivered by any physiologically acceptable mode of administration, including, but not limited to: oral administration, parenteral administration, nasal administration, rectal administration, intraperitoneal administration, intravascular administration, subcutaneous administration, percutaneous administration, inhalation administration, etc. In some embodiments, pharmaceutical compositions for therapeutic use may be formulated in the form of freeze-dried preparations or aqueous solutions for storage by mixing reagents of the desired purity with pharmaceutically acceptable carriers, excipients, etc., as appropriate.

In some embodiments, the pharmaceutical composition is used to prevent or treat a tumor (such as malignant tumor) or an immune disease. In some embodiments, the tumor (e.g., malignant tumor) is a tumors with high CD24 expression on the surface of the tumor cell, including, but not limited to, lung, breast, ovarian, lymphatic, colorectal, ovarian, liver, cervical, kidney, prostate, esophageal, bladder or nasopharyngeal cancers. In some embodiments, the immune disease is rheumatoid arthritis or systemic lupus erythematosus.

In the eighth aspect, the present application provides a use of the anti-CD24 antibody or antigen-binding fragment thereof in the first to second aspects or the nucleic acid molecule in the third aspect or the vector in the fourth aspect or the host cells in the fifth aspect or the immunoconjugate in the sixth aspect in the manufacture of a medicament for the prevention or treatment of a tumor (e.g., malignant tumor) or an immune disease. In some embodiments, the tumor (e.g., malignant tumor) is a tumor with high CD24 expression on the surface of the tumor cell, including, but not limited to, lung, breast, ovarian, lymphatic, colorectal, ovarian, liver, cervical, kidney, prostate, esophageal, bladder or nasopharyngeal cancers. In some embodiments, the immune disease is rheumatoid arthritis or systemic lupus erythematosus.

In the ninth aspect, the present application provides a method of treating a tumor (e.g., malignant tumor) or an immune disease in an individuals, including administering to the individual in need thereof a therapeutic effective amount of the anti-CD24 antibody or antigen-binding fragment thereof as described in the first to second aspects, the immunoconjugate as described in the sixth aspect or the pharmaceutical composition as described in the seventh aspect. In some embodiments, the tumor (e.g., malignant tumor) is a tumor with high CD24 expression on the surface of the tumor cell, including, but not limited to, lung, breast, ovarian, lymphatic, colorectal, ovarian, liver, cervical, kidney, prostate, esophageal, bladder or nasopharyngeal cancers. In some embodiments, the immune disease is rheumatoid arthritis or systemic lupus erythematosus.

In the tenth aspect, the present application provides a method for preparing the anti-CD24 antibody or antigen-binding fragment thereof as described in the first to second aspects, including a step for culturing the host cell as described in the fifth aspect. In some embodiments, the method further includes a step to isolate the anti-CD24 antibody or antigen-binding fragment thereof.

It should be understood that the above detailed description is intended only to give those skilled in the art a clearer understanding of the content of the present application and is not intended to restrict it in any way. Those skilled in the art are capable of making various modifications and variations to the embodiments.

### EXAMPLES

The present application is further elaborated in combination with specific embodiments. It should be understood that these examples are intended only to illustrate but not to limit the scope of the present application.

The CDR grafting of antibody heavy and light chains as described in the present application and the screening of PCR introduced site mutations are completed by gene recombination technology and immunological technology based on antigen-antibody interaction. For specific experimental methods and procedures, please refer to the third edition of "Molecular Cloning" (Joseph Sambrook, Science Press, August 1, 2002) and similar experimental manuals.

### Example 1. Hybridoma preparation and screening

### 1. Mouse immunization

BALB/C female mice (6-8 weeks old, purchased from Chengdu Dossy Experimental Animals Co., LTD.) were immunized with standard peritoneal immunization or plantar immunization. 10µg/mouse was injected every 2 to 3 weeks. All mice were initially immunized with an equal volume of CFA (SIGMA item number: SLBW7430) mixed and emulsified with antigen, and IFA (SIGMA, item number: F5506) is used to mix and emulsify the antigen before sprint immunization (about the second immunization to the fourth immunization) to form a state of water-in-oil, and the emulsion is completed when it is added dropwise into water and gently shaken without dispersion. The antigen used was commercially purchased recombinant human CD24 Fc chimeric protein (item No. : 5247-CD-050, https://www.rndsystems.com/cn/products/recombinant-human-cd24-fc-chimera-protein-cf_5247-cd). The antigen used for the sprint immunization of mice before hybridoma fusion was formulated with PBS (20 µg/mouse, 100 µl/mouse).

### 2. Hybridoma fusion and culturing

For mice immunized with antigen, the mice were given sprint immunization three days before hybridoma fusion. The mice were killed by twisting their necks, and the mice were soaked in 70% ethanol for 5min for disinfection. Spleen and lymph nodes were extracted, and the spleen and lymph nodes were properly ground. After grinding, the cells were filtered through a 70 µm cell filter, and the cell suspension was transferred into a sterile centrifuge tube at 2000 rpm for 5 min at room temperature. After centrifugation, the supernatant was discarded, and the B lymphocytes were resuspend with 5 ml DMEM (GIBCO, item number: 12491023/10 × 500 mL), and set aside for further use. After adding 12 ml erythrocyte lysis solution to spleen B cells, the cells were dispersed, left at room temperature for 8min, and centrifuged at 2000 rpm for 5 min. The supernatant was discarded, and DMEM medium was added for resuspension. The cells were counted separately, and set aside for further use.

P3X63Ag8.653 myeloma cells (item number: ATCC^{®} CRL-1580, purchased from ATCC) or FO myeloma cells (item number: TCM31, purchased from the Chinese Academy of Sciences Cell Bank) were collected and counted. Myeloma cells and B cells (1:1) were mixed homogeneously, and was suspended in the ECF solution, and the cells were counted. The cell density was adjusted to 2 × 10⁶ to 1 × 10⁷ cells/ml by adding ECF solution. The cell suspension was added to the fusion tank and the ECM2001 electrofusion instrument was run. After the operation, the cell suspension was transferred from the fusion tank to a sterile hybridoma fusion medium containing HAT (GIBCO, item number: 20160017), then mixed thoroughly and added to a 96 well plate; incubated at 37 °C in an 8% carbon dioxide incubator.

On the 7th to 12th day after fusion, according to the growth status of the hybridoma, half of the culture supernatant was discarded, and fresh hybridoma medium was added. After 2-3 liquid changes, ELISA or flow cytometry detection was performed when the cells were in the best state. According to the test results, positive hybridoma cells were selected (OD value was 3 times the OD value of negative control or logshift value was 3 times the logshift value of negative control) and expanded the culturing. A second test were performed 2-3 days later, and the positive hybridoma cell lines were expanded for the culturing.

### 3. Identification of IgG subtypes of candidate murine antibodies

Mouse Monoclonal Antibody Isotyping Kit (Roche,cat no.:11493027001) was used to identify the IgG subtypes of candidate murine antibodies. The specific steps are as follows:
The candidate antibody was diluted to 0.1µg/ mL-1 µg/ml using PBS containing 1% BSA, and the 150µl diluted sample was added to the developing tube and incubated at 15°C to 25°C for 30 s. The black end of the test strip was put into the developing tube, and kept for at least 1 min. Then the test strip was removed. Within 5-10min of placement, the indicated position of the blue stripe is the mouse IgG subtype.

### Example 2. Hybridoma monoclonal antibody gene sequencing

The total RNA of hybridoma cells was extracted by Trizol method, and reverse transcription was performed on 5 µg total RNA to obtain cDNA.Then, using cDNA as template, 5 'RACE technology was used for PCR amplification, and the PCR products were linked into the enzyme digestion vector pcDNA3.4, and the linked products were transformed into competent DH5α cells through heat shock, and colony PCR identification was performed, and a small number of plasmids were extracted from the positive clones and sent for sequencing to obtain the variable regions of light and heavy chains of the hybridoma antibody genes, as shown in Table 1.

**Table 1**

| Antibody number | Heavy chain variable region | Light chain variable region |
|---|---|---|
| KH01 | (SEQ ID NO:57) | (SEQ ID NO:72) |
| KH02 | (SEQ ID NO:58) | (SEQ ID NO:73) |
| KH03 | (SEQ ID NO:59) | (SEQ ID NO:74) |
| KH04 | (SEQ ID NO:60) | (SEQ ID NO:75) |
| KH05 | (SEQ ID NO:61) | (SEQ ID NO:76) |
| KH06 | (SEQ ID NO:62) | (SEQ ID NO:76) |
| KH07 | (SEQ ID NO:63) | (SEQ ID NO:77) |
| KH08 | (SEQ ID NO:64) | (SEQ ID NO:78) |
| KH09 | (SEQ ID NO:65) | (SEQ ID NO:79) |
| KH10 | (SEQ ID NO:59) | (SEQ ID NO:80) |
| KH11 | (SEQ ID NO:59) | (SEQ ID NO:81) |
| KH12 | (SEQ ID NO:66) | (SEQ ID NO:82) |
| KH13 | (SEQ ID NO:66) | (SEQ ID NO:83) |
| KH14 | (SEQ ID NO:61) | (SEQ ID NO:84) |
| KH15 | (SEQ ID NO:61) | (SEQ ID NO:85) |
| KH16 | (SEQ ID NO:67) | (SEQ ID NO:86) |
| KH17 | (SEQ ID NO:68) | (SEQ ID NO:87) |
| KH18 | (SEQ ID NO:57) | (SEQ ID NO:88) |
| KH19 | (SEQ ID NO:61) | (SEQ ID NO:89) |
| KH20 | (SEQ ID NO:61) | (SEQ ID NO:76) |
| KH21 | (SEQ ID NO:57) | (SEQ ID NO:90) |
| KH22 | (SEQ ID NO:69) | (SEQ ID NO:76) |
| KH23 | (SEQ ID NO:57) | (SEQ ID NO:76) |
| KH24 | (SEQ ID NO:69) | (SEQ ID NO:90) |
| KH25 | (SEQ ID NO:59) | (SEQ ID NO:76) |
| KH26 | (SEQ ID NO:59) | (SEQ ID NO:91) |
| KH27 | (SEQ ID NO:70) | (SEQ ID NO:91) |
| KH28 | (SEQ ID NO:70) | (SEQ ID NO:80) |
| KH29 | (SEQ ID NO:71) | (SEQ ID NO:92) |
| KH30 | (SEQ ID NO:57) | (SEQ ID NO:92) |
| KH31 | (SEQ ID NO:67) | (SEQ ID NO:92) |
| KH32 | (SEQ ID NO:57) | (SEQ ID NO:93) |
| KH33 | (SEQ ID NO:57) | (SEQ ID NO:94) |

Among them, the heavy chain constant region of KH01-04, KH08-13, KH16-18, KH21, KH23 and KH32-33 murine antibodies is mouse IgG2a, and the amino acid sequence thereof is set forth in SEQ ID NO:95. The constant heavy chain region of murine antibodies KH05, KH07, KH14-15, KH19-20, KH22, KH24-31 is mouse IgG2b, and the amino acid sequence thereof is set forth in SEQ ID NO:96. The light chain constant region of KH01-33 murine antibodies is the mouse κ type light chain constant region, and the amino acid sequence is shown as SEQ ID NO:97.

### Example 3. Flow cytometry antibody binding experiment

The binding ability of the antibody to engineered cells expressing high CD24, such as high CD24-expressing CHO engineered cells/293 cells, and tumor cells, such as human lung cancer cells (A549 cells), human breast cancer cells (MCF-7, Ovcar-3 cells), human B-cell lymphoma cells, and human colorectal cancer cells, was detected by flow cytometry. 5 × 10⁶/ml cells were mixed with 5 µ g/ml of the above murine antibody in a ratio of 20 µl:20 µ l in a 96-well plate. Positive and negative control wells were set for each cell type. After incubating at room temperature for 1 hour, the cells were washed once with FACS buffer, the supernatant was discarded, and 20 µ l/well of 5 µ g/ml PE Goat Anti-Mouse IgG was added. After incubating at room temperature for 20 minutes, the cells were washed once with FACS buffer, the supernatant was discarded, and the cells were resuspended in FACS buffer and analyzed using a flow cytometer (BD FACS Celesta). The results showed that the antibody of the present application had good binding ability to the engineered cells or tumor cells expressing high CD24, such as high CD24-expressing CHO engineered cells, 293 engineered cells, human lung cancer cells (A549 cells), human breast cancer cells (MCF-7, Ovcar-3 cells), human B-cell lymphoma cells, and human colorectal cancer cells.

### Example 4. Phagocytosis of A549 tumor cells by M2 primary macrophages induced by human PBMC

This example evaluates the macrophage-mediated phagocytosis activity induced by anti-CD24 antibodies. The test principle is as follows: CD24 on tumor cells (for example, A549 in this example and MCF-7 in example 5) binds to Siglec10 on macrophages to form a "don't eat me" signal, thereby inhibiting macrophages from phagocytosing tumor cells. CD24 antibody can block the binding of CD24 and Siglec10 to block the "don't eat me" signal and stimulate the phagocytosis of macrophages.

Human peripheral blood monouclear cells were prepared to induce M2 primary macrophages for 7 days, and the cell density of macrophage suspension was adjusted to 0.7×10⁶ cells/ml, and the cells were set aside for further use. At the same time, A549-GFP-2 (self-made) tumor cells were prepared and the cell density of tumor cell suspension was adjusted to 1.4×10⁶ cells/ml. Then the macrophages and tumor cells were co-incubated: 50 µl macrophage suspension, 50 µl tumor cell suspension, 50 µl negative control antibody (mouse IgG) and the murine antibody prepared by the above example were added to each well of the 96-well plate (the final concentration of the antibody was 5 µg/ml prepared with PBS), and mixed homogeniously. The plates were incubated in a 5% CO₂ cell incubator at 37 °C for 2 h. After incubation, centrifugation was performed to remove supernatant; 50 µl 2 µg/ml anti-human BV421-anti-human CDllb antibody was added to each well, and the cells were resuspended and incubated at room temperature for 30min. Washing: 200 µl FACS buffer was added to each well to resuspend cells, then centrifuged to remove supernatant; 100µl 2µg/ml 7-AAD dye solution was added to resuspend cells. On-board detection was performed by BD FACS Celestra ^{™} (JC345). The results are shown in Table 2.

**Table 2**

| Antibody number | Phagocytosis rate % |
|---|---|
| KH01 | 11.80 |
| KH02 | 10.30 |
| KH03 | 11.95 |
| KH04 | 12.75 |
| KH05 | 10.45 |
| KH06 | 10.90 |
| KH07 | 10.05 |
| KH10 | 12.05 |
| KH12 | 11.05 |
| KH14 | 11.25 |
| KH16 | 12.35 |
| KH19 | 10.35 |
| KH21 | 10.15 |
| KH25 | 11.00 |
| KH27 | 10.60 |
| KH29 | 8.95 |
| KH33 | 18.30 |
| IgG | 2.7 |

### Example 5. Phagocytosis of MCF-7 tumor cells by M2 macrophages induced by human PBMC

This example evaluates the macrophage-mediated phagocytosis activity mediated by anti-CD24 antibodies. Human peripheral blood monouclear cells were prepared to induce M2 primary macrophages for 7 days, and the cell density of macrophage suspension was adjusted to 0.7×10⁶ cells /ml, and the cells were set aside for further use. Meanwhile, MCF-7 luciferase GFP (self-made) tumor cells were prepared, and the cell density of tumor cell suspension was adjusted to 1.4×10⁶ cells/ml. Then the macrophages and tumor cells were co-incubated: 50 µl macrophage cell suspension, 50 µl tumor cell suspension, 50 µl negative control antibody (mouse IgG) and the murine antibody prepared from the above example were added to each well of the 96-well plate (the antibody was prepared with PBS with a final concentration of 5µg/ml), and mixed homogeniously. The plates were incubated in a 5% CO₂ cell incubator at 7°C for 2h. After incubation, centrifugation was performed to remove supernatant; 50 µl 2 µg/ml BV421- anti-human CD11b antibody was added to each well, and the cells were suspended and incubated at room temperature for 30min. Washing: 200µl FACS buffer was added to each well to resuspend cells, then centrifuged to remove supernatant; 100µl 2µg/ml 7-AAD dye solution was added to resuspend cells. On-board detection was performed by BD FACS Celestra^{™} (JC345). The results are shown in Table 3.

**Table 3**

| Number | Phagocytosis rate (%) |
|---|---|
| KH01 | 6.2 |
| KH02 | 3.0 |
| KH03 | 3.6 |
| KH05 | 3.2 |
| KH06 | 4.3 |
| KH14 | 3.4 |
| KH16 | 6.8 |
| KH25 | 6.5 |
| IgG | 1.3 |

### Example 6. Construction of chimeric antibody

According to the variable region gene sequence of mouse hybridoma antibody and the constant region of human light and heavy chain, the primers were designed to amplify the variable region gene sequence of mouse hybridoma antibody by PCR, and the chimeric antibody Q fragment was obtained. The human light chain constant region (hIgkCL) and human heavy chain constant region (hIgG1CH) were amplified by PCR to obtain chimeric antibody H fragment. The Q fragment and the H fragment were combined into the expression vector of enzyme digestion vector pcDNA3.4 by homologous recombination. The correctly sequenced plasmids were transfected into expiCHO or HEK293 cells for protein expression. The cells were cultured for about 7-10 days, and the cell supernatant was collected by centrifugation. The cell supernatant was purified by affinity using an AKTA protein purifier to obtain chimeric antibody proteins.

### Example 7. Humanization of antibodies

Since murine antibodies are easy to cause human HAMA reaction, in order to reduce the immunogenicity of murine antibodies in human body, it is necessary to humanize the murine antibodies. In this example, multiple candidate molecules were humanized by CDR grafting, framework reorganization, key amino acid reverting mutation design, combined with DS (Discovery Studio) software. Firstly, the light and heavy chain CDR regions of murine antibody were grafted into the FR region of human germline gene with highly similar sequences through NCBI database comparison, and the variable regions of humanized antibody were obtained by gene synthesis method. The humanization method of antibody with the assistance of DS software was to input the amino acid sequence of the variable region of murine antibody sequenced by hybridoma antibody into DS software to analyze the CDR region and FR region. The homologous modeling method was used to model the antibody. After the modeling was completed, force field was added and the humanization module was applied to humanize the antibody, and four humanized sequences were obtained, which were as follows: Germline, Best_Single_Mutations, Germline_Substitutions, Frequent_Residue_Substitutions. Based on the Germline sequences obtained, the amino acid sites were selected by performing reverting mutation: DS software was used to select the sequences within the 5 Å range of the CDR region of the antibody and the contact surface of the light and heavy chains of the antibody, and the human amino acids in this region were reverted to murine amino acids, and the variable regions of the humanized antibody were obtained by gene synthesis.

After the humanized antibody variable region was obtained by the above mentioned method, the light and heavy chain constant region (hIgkCL and hIgG1CH) and the corresponding antibody variable region were spliced and connected into the pcDNA3.4 expression vector by homologous recombination method, and the correct antibody light and heavy chain expression plasmid was obtained by colony PCR and sequencing. Then the light and heavy chain plasmids were transfected into expiCHO or HEK293 cells for expression. 7-10 days later, the cell supernatant was purified by affinity using AKTA protein purifier to obtain full-length humanized antibody proteins, as shown in Table 4.

**Table 4**

| Antibody number | Heavy chain variable region | Light chain variable region |
|---|---|---|
| KH34 | SEQ ID NO:101 | SEQ ID NO:137 |
| KH35 | SEQ ID NO:98 | SEQ ID NO:144 |
| KH36 | SEQ ID NO:99 | SEQ ID NO:122 |
| KH37 | SEQ ID NO:114 | SEQ ID NO:143 |
| KH38 | SEQ ID NO:98 | SEQ ID NO:142 |
| KH39 | SEQ ID NO:101 | SEQ ID NO:124 |
| KH40 | SEQ ID NO:98 | SEQ ID NO:122 |
| KH41 | SEQ ID NO:99 | SEQ ID NO:121 |
| KH42 | SEQ ID NO:98 | SEQ ID NO:124 |
| KH43 | SEQ ID NO:98 | SEQ ID NO:137 |
| KH44 | SEQ ID NO:98 | SEQ ID NO:121 |
| KH45 | SEQ ID NO:101 | SEQ ID NO:121 |
| KH46 | SEQ ID NO:101 | SEQ ID NO:133 |
| KH47 | SEQ ID NO:99 | SEQ ID NO:124 |
| KH48 | SEQ ID NO:99 | SEQ ID NO:134 |
| KH49 | SEQ ID NO:111 | SEQ ID NO:136 |
| KH50 | SEQ ID NO:98 | SEQ ID NO:134 |
| KH51 | SEQ ID NO:98 | SEQ ID NO:133 |
| KH52 | SEQ ID NO:99 | SEQ ID NO:133 |
| KH53 | SEQ ID NO:101 | SEQ ID NO:122 |
| KH54 | SEQ ID NO:118 | SEQ ID NO:146 |
| KH55 | SEQ ID NO:99 | SEQ ID NO:137 |
| KH56 | SEQ ID NO:115 | SEQ ID NO:122 |
| KH57 | SEQ ID NO:117 | SEQ ID NO:121 |
| KH58 | SEQ ID NO:116 | SEQ ID NO:145 |
| KH59 | SEQ ID NO:104 | SEQ ID NO:125 |
| KH60 | SEQ ID NO:105 | SEQ ID NO:123 |
| KH61 | SEQ ID NO:110 | SEQ ID NO:126 |
| KH62 | SEQ ID NO:109 | SEQ ID NO:123 |
| KH63 | SEQ ID NO:110 | SEQ ID NO:123 |
| KH64 | SEQ ID NO:104 | SEQ ID NO:130 |
| KH65 | SEQ ID NO:109 | SEQ ID NO:126 |
| KH66 | SEQ ID NO:108 | SEQ ID NO:130 |
| KH67 | SEQ ID NO:105 | SEQ ID NO:126 |
| KH68 | SEQ ID NO:113 | SEQ ID NO:141 |
| KH69 | SEQ ID NO:108 | SEQ ID NO:125 |
| KH70 | SEQ ID NO:103 | SEQ ID NO:139 |
| KH71 | SEQ ID NO:107 | SEQ ID NO:140 |
| KH72 | SEQ ID NO:106 | SEQ ID NO:140 |
| KH73 | SEQ ID NO:106 | SEQ ID NO:129 |
| KH74 | SEQ ID NO:107 | SEQ ID NO:129 |
| KH75 | SEQ ID NO:107 | SEQ ID NO:128 |
| KH76 | SEQ ID NO:103 | SEQ ID NO:128 |
| KH77 | SEQ ID NO:103 | SEQ ID NO:140 |
| KH78 | SEQ ID NO:103 | SEQ ID NO:129 |
| KH79 | SEQ ID NO:120 | SEQ ID NO:139 |
| KH80 | SEQ ID NO:106 | SEQ ID NO:128 |
| KH81 | SEQ ID NO:112 | SEQ ID NO:147 |
| KH82 | SEQ ID NO:112 | SEQ ID NO:138 |
| KH83 | SEQ ID NO:119 | SEQ ID NO:138 |
| KH84 | SEQ ID NO:100 | SEQ ID NO:127 |
| KH85 | SEQ ID NO:102 | SEQ ID NO:127 |
| KH86 | SEQ ID NO:100 | SEQ ID NO:135 |
| KH87 | SEQ ID NO:102 | SEQ ID NO:131 |
| KH88 | SEQ ID NO:100 | SEQ ID NO:132 |
| KH89 | SEQ ID NO:102 | SEQ ID NO:135 |
| KH90 | SEQ ID NO:102 | SEQ ID NO:132 |
| KH91 | SEQ ID NO:100 | SEQ ID NO:131 |

Among them, the antibody heavy chain constant region of KH34-KH91 is human IgG1, and the amino acid sequence thereof is set forth in SEQ ID NO:148; the antibody light chain constant region is κ type light chain constant region, and the amino acid sequence thereof is set forth in SEQ ID NO:151.

### Example 8: Animal efficacy study

### 1.1 Modeling of animal efficacy

Animal: BALB/c nude mouse, 5 weeks old, female, purchased from GemPharmatech.

Modeling: Subcutaneous (left dorsal) inoculation of nude mice with A549 cell suspension, 5×10⁷ cells/ml, 200µl/mouse.

### 1.2 Sample Preparation

After the antibody sample was prepared with PBS, 1.6 ml/aliquot was divided.

### 1.3 Drug delivery

The next day of inoculation, 100 µl/animal was administered in the tail vein, twice a week, a total of 6 times, 10 animals/group. This example tests the murine antibody in example 2 and the humanized antibody in example 7.

### 1.4 Experimental monitoring

Monitoring index 1: 2 times/week after the first administration, the long side (L) and short side (W) of the tumor were measured by a vernier caliper and calculated according to the following formula: L×W²/2 to calculate the tumor volume.

Monitoring index 2: 3 weeks after the first administration, the animals were killed, the subcutaneous tumor was dissected, and the tumor weight was measured.

### 1.5 Experimental Results

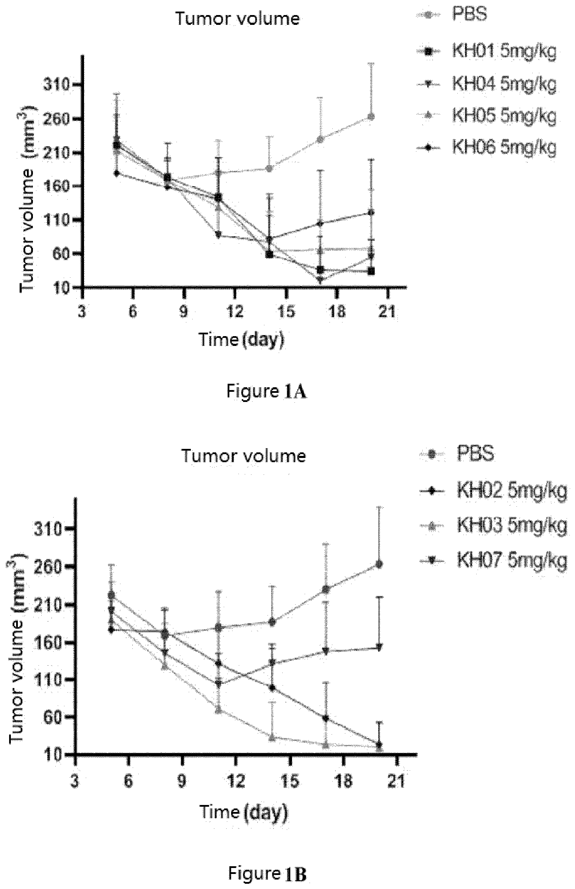

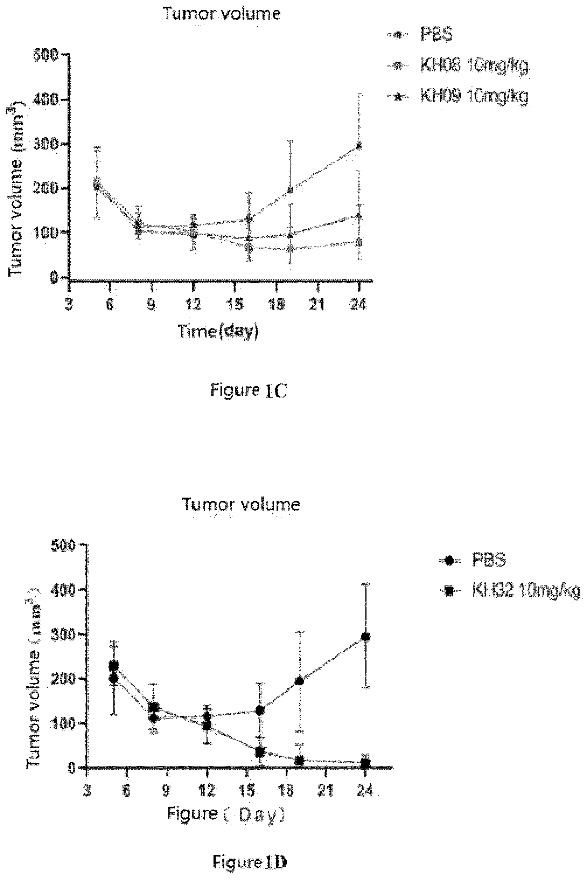

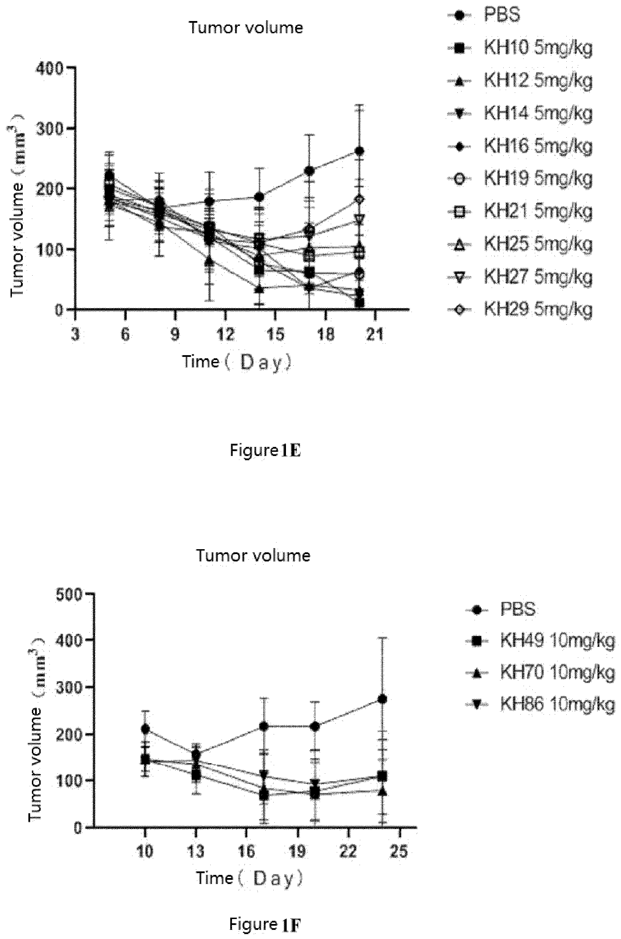

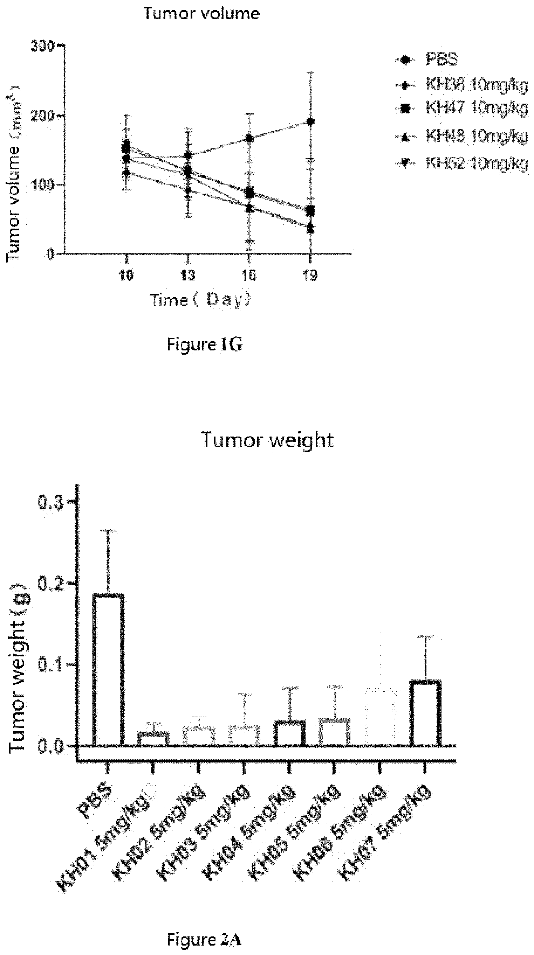

Monitoring index 1: Tumor volume is shown in Figure 1A-1G. As can be seen from the statistical graph of continuous monitoring of subcutaneous tumor volume, the tumor volume of each administration group decreased significantly as compared with the control group (PBS).

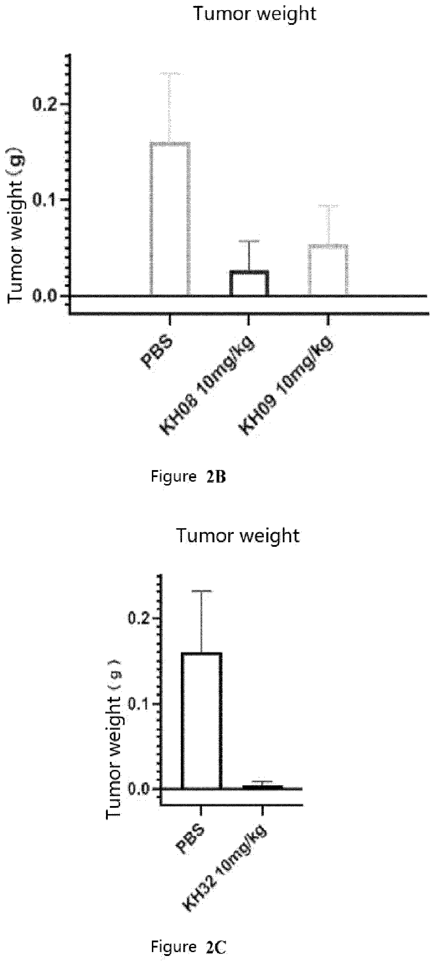

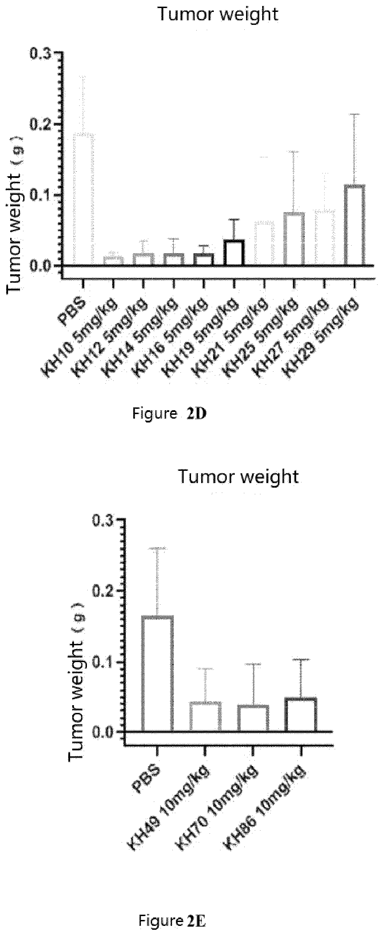

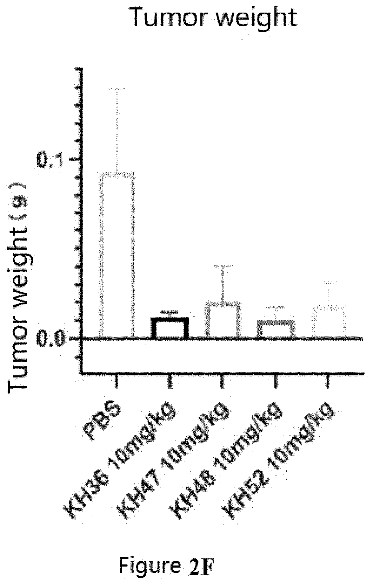

Monitoring index 2: The results of tumor weighing after subcutaneous tumor dissection are shown in Figure 2A-2F. It can be seen from the figures that the tumor weight of each administration group decreased significantly as compared with the control group (PBS).

## Claims

1. An anti-CD24 antibody or an antigen-binding fragment thereof, wherein the anti-CD24 antibody or antigen-binding fragment thereof comprises a heavy chain variable region and a light chain variable region, wherein the heavy chain variable region comprises heavy chain CDR1 (HCDR1), heavy chain CDR2 (HCDR2) and heavy chain CDR3 (HCDR3), the light chain variable region comprises light chain CDR1 (LCDR1), light chain CDR2 (LCDR2) and light chain CDR3 (LCDR3), wherein:
the amino acid sequence of HCDR1 comprises X₁GYX₂WH (SEQ ID NO: 152), wherein X₁ is R, N or S, X₂ is S or T; or the amino acid sequence of HCDR1 is TYGVS (SEQ ID NO:5);
the amino acid sequence of HCDR2 comprises X₁X₂X₃X₄X₅GX₆TX₇YX₈X₉X₁₀LX₁₁X₁₂ (SEQ ID NO:153), wherein X₁ is Y, V or F, X₂ is I or T, X₃ is H, W or Q; X₄ is H, G, Y or F; X₅ is R, S or D; X₆ is S, T, N or A; X₇ is N or K; X₈ is N or H; X₉ is P or S; X₁₀ is S or A; X₁₁ is K or I; X₁₂ is S or N;
the amino acid sequence of HCDR3 comprises GX₁X₂X₃X₄X₅DX₆ (SEQ ID NO:154), wherein X₁ is S or T: X₂ is N, D or T; X₃ is S, R, W, G or T; X₄ is A, S or G; X₅ is F or L; X₆ is Y or H; or the amino acid sequence of HCDR3 is YYGYDFFAY (SEQ ID NO:25) or YYGYDFFPY (SEQ ID NO:26);
the amino acid sequence of LCDR1 comprises X₁SSQSLLYSX₂X₃QKNYX₄X₅ (SEQ ID NO:155), wherein X₁ is K or Q, X₂ is N, S or K, X₃ is N or D; X₄ is L or S; X₅ is A, T or V; or the amino acid sequence of LCDR1 is RASQDISNYLN (SEQ ID NO:35), SASSSISYMH (SEQ ID NO:36), SASSSLSYMH (SEQ ID NO:37), SASSSVSSSYLY (SEQ IDNO:38) or SASSSVSYMH (SEQ ID NO:39);
the amino acid sequence of LCDR2 comprises X₁X₂SX₃X₄X₅S (SEQ ID NO:156), wherein X₁ is W, L, D, S or Y; X₂ is A or T; X₃ is T, K, N or R; X₄ is R, K or L; X₅ is E, A, Y or H;
the amino acid sequence of LCDR3 comprises X₁QX₂X₃X₄X₅PX₆X₇ (SEQ ID NO:157), wherein X₁ is Q, H or F; X₂ is Y, G, R or W; X₃ is Y, S, N or F; X₄ is I, G, S, T or L; X₅ is Y or L; X₆ is L, F, C, P, H or M; X₇ is T or S;
wherein the sequences of HCDR1-3 and LCDR1-3 are defined according to Kabat.

2. The anti-CD24 antibody or antigen-binding fragment thereof according to claim 1, wherein the HCDR1 has any one amino acid sequence of SEQ ID NO:1-5, HCDR2 has any one amino acid sequence of SEQ ID NO:6-18, HCDR3 has any one amino acid sequence in SEQ ID NO:19-26; LCDR1 has any of the amino acid sequences in SEQ ID NO:27-39, LCDR2 has any of the amino acid sequences in SEQ ID NO:40-47, LCDR3 has any of the amino acid sequences in SEQ ID NO:48-56.

3. The anti-CD24 antibody or antigen-binding fragment thereof according to claim 1, wherein:
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 35, SEQ ID NO: 47, SEQ ID NO: 53, respectively;
the amino acid sequences of HCDR1-HCDR3 are SEQ ID NO: 1, SEQ ID NO: 17, SEQ ID NO: 21, respectively, and the amino acid sequences of LCDR1-LCDR3 are SEQ ID NO: 27, SEQ ID NO: 43, SEQ ID NO: 55, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 28, SEQ ID NO: 45, SEQ ID NO: 48, respectively;
the amino acid sequences of HCDR1-HCDR3 are SEQ ID NO: 1, SEQ ID NO: 6, SEQ ID NO: 19, respectively, and LCDR1-LCDR3 are SEQ ID NO: 28, SEQ ID NO: 41, SEQ ID NO: 48, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 13, SEQ ID NO: 20, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 16, SEQ ID NO: 24, respectively, and the amino acid sequences of LCDR1-LCDR3 are SEQ ID NO: 34, SEQ ID NO: 40, SEQ ID NO: 49, respectively;
the amino acid sequences of HCDR1-HCDR3 are SEQ ID NO: 5, SEQ ID NO: 9, SEQ ID NO: 25, respectively, and the amino acid sequences of LCDR1-LCDR3 are SEQ ID NO: 37, SEQ ID NO: 42, SEQ ID NO: 50, respectively;
the amino acid sequences of HCDR1-HCDR3 are SEQ ID NO: 5, SEQ ID NO: 9, SEQ ID NO: 26, respectively, and the amino acid sequences of LCDR1-LCDR3 are SEQ ID NO: 39, SEQ ID NO: 42, SEQ ID NO: 50, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 32, SEQ ID NO: 40, SEQ ID NO: 48, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 1, SEQ ID NO: 10, SEQ ID NO: 21, respectively, and the amino acid sequences of LCDR1-LCDR3 are SEQ ID NO: 31, SEQ ID NO: 46, SEQ ID NO: 49, respectively;
the amino acid sequences of HCDR1-HCDR3 are SEQ ID NO: 1, SEQ ID NO: 10, SEQ ID NO: 21, respectively, and the amino acid sequences of LCDR1-LCDR3 are SEQ ID NO: 27, SEQ ID NO: 43, SEQ ID NO: 56, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 30, SEQ ID NO: 40, SEQ ID NO: 49, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 30, SEQ ID NO: 40, SEQ ID NO: 49, respectively;
the amino acid sequences of HCDR1-HCDR3 are SEQ ID NO: 3, SEQ ID NO: 12, SEQ ID NO: 22, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 3, SEQ ID NO: 18, SEQ ID NO: 22, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 31, SEQ ID NO: 40, SEQ ID NO: 54, respectively;
the amino acid sequences of HCDR1-HCDR3 are SEQ ID NO: 2, SEQ ID NO: 8, SEQ ID NO: 20, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 3, SEQ ID NO: 15, SEQ ID NO: 20, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 3, SEQ ID NO: 15, SEQ ID NO: 20, respectively, and the amino acid sequences of LCDR1-LCDR3 are SEQ ID NO: 28, SEQ ID NO: 41, SEQ ID NO: 48, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 27, SEQ ID NO: 40, SEQ ID NO: 48, respectively;
the amino acid sequences of HCDR1-HCDR3 are SEQ ID NO: 1, SEQ ID NO: 6, SEQ ID NO: 19, respectively, and the amino acid sequences of LCDR1-LCDR3 are SEQ ID NO: 33, SEQ ID NO: 40, SEQ ID NO: 49, respectively;
the amino acid sequences of HCDR1-HCDR3 are SEQ ID NO: 4, SEQ ID NO: 11, SEQ ID NO: 23, respectively, and the amino acid sequences of LCDR1-LCDR3 are SEQ ID NO: 33, SEQ ID NO: 40, SEQ ID NO: 49, respectively;
the amino acid sequences of HCDR1-HCDR3 are SEQ ID NO: 4, SEQ ID NO: 11, SEQ ID NO: 23, respectively, and the amino acid sequences of LCDR1-LCDR3 are SEQ ID NO: 32, SEQ ID NO: 40, SEQ ID NO: 48, respectively;
the amino acid sequences of HCDR1-HCDR3 are SEQ ID NO: 1, SEQ ID NO: 14, SEQ ID NO: 19, respectively, and the amino acid sequences of LCDR1-LCDR3 are SEQ ID NO: 29, SEQ ID NO: 40, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 29, SEQ ID NO: 40, SEQ ID NO: 48, respectively;
the amino acid sequences of HCDR1-HCDR3 are SEQ ID NO: 3, SEQ ID NO: 12, SEQ ID NO: 22, respectively, and the amino acid sequences of LCDR1-LCDR3 are SEQ ID NO: 29, SEQ ID NO: 40, SEQ ID NO: 48, respectively;
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 38, SEQ ID NO: 44, SEQ ID NO: 52, respectively; or
the amino acid sequences of HCDR1-HCDR3 are SEQ ID NO: 1, SEQ ID NO: 7, SEQ ID NO: 19, respectively, and the amino acid sequences of LCDR1-LCDR3 are SEQ ID NO: 36, SEQ ID NO: 42, SEQ ID NO: 51, respectively.

4. The anti-CD24 antibody or antigen-binding fragment thereof according to any one of the preceding claims, wherein the heavy chain variable region has the amino acid sequence as set forth in any one of SEQ ID NO: 57-71 and 98-120 or an amino acid sequence with at least 80%, at least 90%, at least 95% or at least 99% homology to the amino acid sequence as set forth in any one of SEQ ID NO:57-71 and 98-120.

5. The anti-CD24 antibody or antigen-binding fragment thereof according to any one of the preceding claims, wherein the light chain variable region has the amino acid sequence as set forth in any one of SEQ ID NO: 72-94 and 121-147 or an amino acid sequence with at least 80%, at least 90%, at least 95% or at least 99% homology to the amino acid sequence as set forth in any one of 72-94 and 121-147.

6. The anti-CD24 antibody or antigen-binding fragment thereof according to any one of the preceding claims, wherein:
the amino acid sequence of the heavy chain variable region is SEQ ID NO: 57, and the amino acid sequence of the light chain variable region is SEQ ID NO: 72;
the amino acid sequence of the heavy chain variable region is SEQ ID NO: 58, and the amino acid sequence of the light chain variable region is SEQ ID NO: 73;
the amino acid sequence of the heavy chain variable region is SEQ ID NO: 59, and the amino acid sequence of the light chain variable region is SEQ ID NO: 74;
the amino acid sequence of the heavy chain variable region is SEQ ID NO: 60, and the amino acid sequence of the light chain variable region is SEQ ID NO: 75;
the amino acid sequence of the heavy chain variable region is SEQ ID NO: 61, and the amino acid sequence of the light chain variable region is SEQ ID NO: 76;
the amino acid sequence of the heavy chain variable region is SEQ ID NO: 62, and the amino acid sequence of the light chain variable region is SEQ ID NO: 76;
the amino acid sequence of the heavy chain variable region is SEQ ID NO: 63, and the amino acid sequence of the light chain variable region is SEQ ID NO: 77;
the amino acid sequence of the heavy chain variable region is SEQ ID NO: 64, and the amino acid sequence of the light chain variable region is SEQ ID NO: 78;
the amino acid sequence of the heavy chain variable region is SEQ ID NO: 65, and the amino acid sequence of the light chain variable region is SEQ ID NO: 79;
the amino acid sequence of the heavy chain variable region is SEQ ID NO: 59, and the amino acid sequence of the light chain variable region is SEQ ID NO: 80;
the amino acid sequence of the heavy chain variable region is SEQ ID NO: 59, and the amino acid sequence of the light chain variable region is SEQ ID NO: 81;
the amino acid sequence of the heavy chain variable region is SEQ ID NO: 66, and the amino acid sequence of the light chain variable region is SEQ ID NO: 82;
the amino acid sequence of the heavy chain variable region is SEQ ID NO: 66, and the amino acid sequence of the light chain variable region is SEQ ID NO: 83;
the amino acid sequence of the heavy chain variable region is SEQ ID NO: 61, and the amino acid sequence of the light chain variable region is SEQ ID NO: 84;
the amino acid sequence of the heavy chain variable region is SEQ ID NO: 61, and the amino acid sequence of the light chain variable region is SEQ ID NO: 85;
the amino acid sequence of the heavy chain variable region is SEQ ID NO: 67, and the amino acid sequence of the light chain variable region is SEQ ID NO: 86;
the amino acid sequence of the heavy chain variable region is SEQ ID NO: 68, and the amino acid sequence of the light chain variable region is SEQ ID NO: 87;
the amino acid sequence of the heavy chain variable region is SEQ ID NO: 57, and the amino acid sequence of the light chain variable region is SEQ ID NO: 88;
the amino acid sequence of the heavy chain variable region is SEQ ID NO: 61, and the amino acid sequence of the light chain variable region is SEQ ID NO: 89;
the amino acid sequence of the heavy chain variable region is SEQ ID NO: 61, and the amino acid sequence of the light chain variable region is SEQ ID NO: 76;
the amino acid sequence of the heavy chain variable region is SEQ ID NO: 57, and the amino acid sequence of the light chain variable region is SEQ ID NO: 90;
the amino acid sequence of the heavy chain variable region is SEQ ID NO: 69, and the amino acid sequence of the light chain variable region is SEQ ID NO: 76;
the amino acid sequence of the heavy chain variable region is SEQ ID NO: 57, and the amino acid sequence of the light chain variable region is SEQ ID NO: 76;
the amino acid sequence of the heavy chain variable region is SEQ ID NO: 69, and the amino acid sequence of the light chain variable region is SEQ ID NO: 90;
the amino acid sequence of the heavy chain variable region is SEQ ID NO: 59, and the amino acid sequence of the light chain variable region is SEQ ID NO: 76;
the amino acid sequence of the heavy chain variable region is SEQ ID NO: 59, and the amino acid sequence of the light chain variable region is SEQ ID NO: 91;
the amino acid sequence of the heavy chain variable region is SEQ ID NO: 70, and the amino acid sequence of the light chain variable region is SEQ ID NO: 91;
the amino acid sequence of the heavy chain variable region is SEQ ID NO: 70, and the amino acid sequence of the light chain variable region is SEQ ID NO: 80;
the amino acid sequence of the heavy chain variable region is SEQ ID NO: 71, and the amino acid sequence of the light chain variable region is SEQ ID NO: 92;
the amino acid sequence of the heavy chain variable region is SEQ ID NO: 57, and the amino acid sequence of the light chain variable region is SEQ ID NO: 92;
the amino acid sequence of the heavy chain variable region is SEQ ID NO: 67, and the amino acid sequence of the light chain variable region is SEQ ID NO: 92;
the amino acid sequence of the heavy chain variable region is SEQ ID NO: 57, and the amino acid sequence of the light chain variable region is SEQ ID NO: 93;
the amino acid sequence of the heavy chain variable region is SEQ ID NO: 57, and the amino acid sequence of the light chain variable region is SEQ ID NO: 94;
the amino acid sequence of the heavy chain variable region is SEQ ID NO: 101, and the amino acid sequence of the light chain variable region is SEQ ID NO: 137;
the amino acid sequence of the heavy chain variable region is SEQ ID NO: 98, and the amino acid sequence of the light chain variable region is SEQ ID NO: 144;
the amino acid sequence of the heavy chain variable region is SEQ ID NO: 99, and the amino acid sequence of the light chain variable region is SEQ ID NO: 122;
the amino acid sequence of the heavy chain variable region is SEQ ID NO: 114, and the amino acid sequence of the light chain variable region is SEQ ID NO: 143;
the amino acid sequence of the heavy chain variable region is SEQ ID NO: 98, and the amino acid sequence of the light chain variable region is SEQ ID NO: 142;
the amino acid sequence of the heavy chain variable region is SEQ ID NO: 101, and the amino acid sequence of the light chain variable region is SEQ ID NO: 124;
the amino acid sequence of the heavy chain variable region is SEQ ID NO: 98, and the amino acid sequence of the light chain variable region is SEQ ID NO: 122;
the amino acid sequence of the heavy chain variable region is SEQ ID NO: 99, and the amino acid sequence of the light chain variable region is SEQ ID NO: 121;
the amino acid sequence of the heavy chain variable region is SEQ ID NO: 98, and the amino acid sequence of the light chain variable region is SEQ ID NO: 124;
the amino acid sequence of the heavy chain variable region is SEQ ID NO: 98, and the amino acid sequence of the light chain variable region is SEQ ID NO: 137;
the amino acid sequence of the heavy chain variable region is SEQ ID NO: 98, and the amino acid sequence of the light chain variable region is SEQ ID NO: 121;
the amino acid sequence of the heavy chain variable region is SEQ ID NO: 101, and the amino acid sequence of the light chain variable region is SEQ ID NO: 121;
the amino acid sequence of the heavy chain variable region is SEQ ID NO: 101, and the amino acid sequence of the light chain variable region is SEQ ID NO: 133;
the amino acid sequence of the heavy chain variable region is SEQ ID NO: 99, and the amino acid sequence of the light chain variable region is SEQ ID NO: 124;
the amino acid sequence of the heavy chain variable region is SEQ ID NO: 99, and the amino acid sequence of the light chain variable region is SEQ ID NO: 134;
the amino acid sequence of the heavy chain variable region is SEQ ID NO: 111, and the amino acid sequence of the light chain variable region is SEQ ID NO: 136;
the amino acid sequence of the heavy chain variable region is SEQ ID NO: 98, and the amino acid sequence of the light chain variable region is SEQ ID NO: 134;
the amino acid sequence of the heavy chain variable region is SEQ ID NO: 98, and the amino acid sequence of the light chain variable region is SEQ ID NO: 133;
the amino acid sequence of the heavy chain variable region is SEQ ID NO: 99, and the amino acid sequence of the light chain variable region is SEQ ID NO: 133;
the amino acid sequence of the heavy chain variable region is SEQ ID NO: 101, and the amino acid sequence of the light chain variable region is SEQ ID NO: 122;
the amino acid sequence of the heavy chain variable region is SEQ ID NO: 118, and the amino acid sequence of the light chain variable region is SEQ ID NO: 146;
the amino acid sequence of the heavy chain variable region is SEQ ID NO: 99, and the amino acid sequence of the light chain variable region is SEQ ID NO: 137;
the amino acid sequence of the heavy chain variable region is SEQ ID NO: 115, and the amino acid sequence of the light chain variable region is SEQ ID NO: 122;
the amino acid sequence of the heavy chain variable region is SEQ ID NO: 117, and the amino acid sequence of the light chain variable region is SEQ ID NO: 121;
the amino acid sequence of the heavy chain variable region is SEQ ID NO: 116, and the amino acid sequence of the light chain variable region is SEQ ID NO: 145;
the amino acid sequence of the heavy chain variable region is SEQ ID NO: 104, and the amino acid sequence of the light chain variable region is SEQ ID NO: 125;
the amino acid sequence of the heavy chain variable region is SEQ ID NO: 105, and the amino acid sequence of the light chain variable region is SEQ ID NO: 123;
the amino acid sequence of the heavy chain variable region is SEQ ID NO: 110, and the amino acid sequence of the light chain variable region is SEQ ID NO: 126;
the amino acid sequence of the heavy chain variable region is SEQ ID NO: 109, and the amino acid sequence of the light chain variable region is SEQ ID NO: 123;
the amino acid sequence of the heavy chain variable region is SEQ ID NO: 110, and the amino acid sequence of the light chain variable region is SEQ ID NO: 123;
the amino acid sequence of the heavy chain variable region is SEQ ID NO: 104, and the amino acid sequence of the light chain variable region is SEQ ID NO: 130;
the amino acid sequence of the heavy chain variable region is SEQ ID NO: 109, and the amino acid sequence of the light chain variable region is SEQ ID NO: 126;
the amino acid sequence of the heavy chain variable region is SEQ ID NO: 108, and the amino acid sequence of the light chain variable region is SEQ ID NO: 130;
the amino acid sequence of the heavy chain variable region is SEQ ID NO: 105, and the amino acid sequence of the light chain variable region is SEQ ID NO: 126;
the amino acid sequence of the heavy chain variable region is SEQ ID NO: 113, and the amino acid sequence of the light chain variable region is SEQ ID NO: 141;
the amino acid sequence of the heavy chain variable region is SEQ ID NO: 108, and the amino acid sequence of the light chain variable region is SEQ ID NO: 125;
the amino acid sequence of the heavy chain variable region is SEQ ID NO: 103, and the amino acid sequence of the light chain variable region is SEQ ID NO: 139;
the amino acid sequence of the heavy chain variable region is SEQ ID NO: 107, and the amino acid sequence of the light chain variable region is SEQ ID NO: 140;
the amino acid sequence of the heavy chain variable region is SEQ ID NO: 106, and the amino acid sequence of the light chain variable region is SEQ ID NO: 140;
the amino acid sequence of the heavy chain variable region is SEQ ID NO: 106, and the amino acid sequence of the light chain variable region is SEQ ID NO: 129;
the amino acid sequence of the heavy chain variable region is SEQ ID NO: 107, and the amino acid sequence of the light chain variable region is SEQ ID NO: 129;
the amino acid sequence of the heavy chain variable region is SEQ ID NO: 107, and the amino acid sequence of the light chain variable region is SEQ ID NO: 128;
the amino acid sequence of the heavy chain variable region is SEQ ID NO: 103, and the amino acid sequence of the light chain variable region is SEQ ID NO: 128;
the amino acid sequence of the heavy chain variable region is SEQ ID NO: 103, and the amino acid sequence of the light chain variable region is SEQ ID NO: 140;
the amino acid sequence of the heavy chain variable region is SEQ ID NO: 103, and the amino acid sequence of the light chain variable region is SEQ ID NO: 129;
the amino acid sequence of the heavy chain variable region is SEQ ID NO: 120, and the amino acid sequence of the light chain variable region is SEQ ID NO: 139;
the amino acid sequence of the heavy chain variable region is SEQ ID NO: 106, and the amino acid sequence of the light chain variable region is SEQ ID NO: 128;
the amino acid sequence of the heavy chain variable region is SEQ ID NO: 112, and the amino acid sequence of the light chain variable region is SEQ ID NO: 147;
the amino acid sequence of the heavy chain variable region is SEQ ID NO: 112, and the amino acid sequence of the light chain variable region is SEQ ID NO: 138;
the amino acid sequence of the heavy chain variable region is SEQ ID NO: 119, and the amino acid sequence of the light chain variable region is SEQ ID NO: 138;
the amino acid sequence of the heavy chain variable region is SEQ ID NO: 100, and the amino acid sequence of the light chain variable region is SEQ ID NO: 127;
the amino acid sequence of the heavy chain variable region is SEQ ID NO: 102, and the amino acid sequence of the light chain variable region is SEQ ID NO: 127;
the amino acid sequence of the heavy chain variable region is SEQ ID NO: 100, and the amino acid sequence of the light chain variable region is SEQ ID NO: 135;
the amino acid sequence of the heavy chain variable region is SEQ ID NO: 102, and the amino acid sequence of the light chain variable region is SEQ ID NO: 131;
the amino acid sequence of the heavy chain variable region is SEQ ID NO: 100, and the amino acid sequence of the light chain variable region is SEQ ID NO: 132;
the amino acid sequence of the heavy chain variable region is SEQ ID NO: 102, and the amino acid sequence of the light chain variable region is SEQ ID NO: 135;
the amino acid sequence of the heavy chain variable region is SEQ ID NO: 102, and the amino acid sequence of the light chain variable region is SEQ ID NO: 132; or
the amino acid sequence of the heavy chain variable region is SEQ ID NO: 100, and the amino acid sequence of the light chain variable region is SEQ ID NO: 131.

7. An anti-CD24 antibody or an antigen-binding fragment thereof, wherein the anti-CD24 antibody or antigen-binding fragment thereof comprises a heavy chain variable region, wherein the heavy chain variable region comprises heavy chain CDR1 (HCDR1), heavy chain CDR2 (HCDR2) and heavy chain CDR3 (HCDR3), wherein:
the amino acid sequence of HCDR1 comprises X₁GYX₂WH (SEQ ID NO:152), wherein X₁ is R, N or S, X₂ is S or T; or the amino acid sequence of HCDR1 is TYGVS (SEQ ID NO:5);
the amino acid sequence of HCDR2 comprises X₁X₂X₃X₄X₅GX₆TX₇YX₈X₉X₁₀LX₁₁X₁₂ (SEQ ID NO:153), wherein X₁ is Y, V or F, X₂ is I or T, X₃ is H, W or Q; X₄ is H, G, Y or F; X₅ is R, S or D; X₆ is S, T, N or A; X₇ is N or K; X₈ is N or H; X₉ is P or S; X₁₀ is S or A; X₁₁ is K or I; X₁₂ is S or N;
the amino acid sequence of HCDR3 comprises GX₁X₂X₃X₄X₅DX₆ (SEQ ID NO:154), wherein X₁ is S or T; Xz is N, D or T; X₃ is S, R, W, G or T; X₄ is A, S or G; X₅ is F or L; X₆ is Y or H; or the amino acid sequence of HCDR3 is YYGYDFFAY (SEQ ID NO:25) or YYGYDFFPY (SEQ ID NO:26);
wherein the sequences of HCDR1-3 are defined according to Kabat.

8. The anti-CD24 antibody or antigen-binding fragment thereof according to any one of the preceding claims, wherein the light chain constant region of the anti-CD24 antibody or antigen-binding fragment thereof is the light chain constant region of a human antibody; preferably, the light chain constant region of the anti-CD24 antibody or antigen-binding fragment thereof is selected from human κ type or λ type light chain constant region; more preferably, the light chain constant region of the anti-CD24 antibody or antigen-binding fragment thereof is the x-type light chain constant region.

9. The anti-CD24 antibody or antigen-binding fragment thereof according to any one of the preceding claims, wherein the heavy chain constant region of the anti-CD24 antibody or antigen-binding fragment thereof is the heavy chain constant region of the human antibody; preferably, the heavy chain constant region of the anti-CD24 antibody or antigen-binding fragment thereof is selected from any one of the human IgD, IgE, IgM, IgA, IgG1, IgG2a, IgG2b, IgG3, and IgG4 types; more preferably, the heavy chain constant region of the antibody or antigen-binding fragment thereof is selected from IgG1, IgG2 or IgG4 type.

10. The anti-CD24 antibody or antigen-binding fragment thereof according to any one of the preceding claims, wherein:
the anti-CD24 antibody is a whole antibody, a single chain antibody (scFv) or a bispecific antibody; and/or
the antigen-binding fragment of the anti-CD24 antibody is Fab, Fab', Fv or F(ab')₂; and/or
the anti-CD24 antibody is a murine antibody, a chimeric antibody, a humanized antibody or a fully human antibody; and/or
the anti-CD24 antibody is a monoclonal antibody.

11. An isolated nucleic acid molecule encoding the anti-CD24 antibody or antigen-binding fragment thereof as claimed in any one of claims 1-10.

12. A vector comprising the nucleic acid molecule as claimed in claim 11; preferably, the vector is an expression vector.

13. An isolated host cell comprising the nucleic acid molecule according to claim 11 or the vector according to claim 12.

14. An immunoconjugate comprising the anti-CD24 antibody or antigen-binding fragment thereof as claimed in any one of claims 1-10 conjugated to a therapeutic agent..

15. The immunoconjugate according to claim 14, wherein the therapeutic agent is an anti-tumor drug, for example, a cytotoxic drug, an immunoenhancer or a radioisotope.

16. An pharmaceutical composition comprising the anti-CD24 antibody or antigen-binding fragment thereof according to any one of claims 1-10 or the immunoconjugate according to claim 14 or 15, and a pharmaceutically acceptable excipient, diluent or carrier.

17. The pharmaceutical composition according to claim 16, which is used for the prevention or treatment of a tumor (e.g., malignant tumor) or an immune disease.

18. The pharmaceutical composition according to claim 17, wherein the tumor (e.g., malignant tumor) is a tumor with high CD24 expression of on the tumor cell surface, e.g., lung, breast, ovarian, lymphatic, colorectal, ovarian, liver, cervical, kidney, prostate, esophageal, bladder or nasopharyngeal cancer.

19. The pharmaceutical composition according to claim 17, wherein the immune disease is rheumatoid arthritis or systemic lupus erythematosus.

20. Use of the anti-CD24 antibody or antigen-binding fragment thereof according to any one of claims 1-10 or the isolated nucleic acid molecule according to claim 11 or the vector according to claim 12 or the isolated host cell according to claim 13 or the immunoconjugate according to claim 14 or 15 in the manufacture of a medicament for the prevention or treatment of a tumor (e.g., malignant tumor) or an immune disease.

21. The use according to claim 20, wherein the tumor (e.g., malignant tumor) is a tumor with high CD24 expression on the tumor cell surface, e.g., lung, breast, ovarian, lymphatic, colorectal, ovarian, liver, cervical, kidney, prostate, esophageal, bladder or nasopharyngeal cancer.

22. The use according to claim 20, wherein the immune disease is rheumatoid arthritis or systemic lupus erythematosus.

23. A method for treating a tumor (e.g., malignant tumor) or an immune disease in an individual, including administering to an individual in need thereof a therapeutically effective amount of the anti-CD24 antibody or antigen-binding fragment thereof according to any one of claims 1-10 or the immunoconjugate according to claim 14 or 15 or the pharmaceutical composition according to claim 16.

24. The method according claim 23, wherein the tumor (e.g., malignant tumor) is a tumor with high CD24 expression on the tumor cell surface, e.g., lung, breast, ovarian, lymphatic, colorectal, ovarian, liver, cervical, kidney, prostate, esophageal, bladder or nasopharyngeal cancer.

25. The method according claim 23, wherein the immune disease is rheumatoid arthritis or systemic lupus erythematosus.

26. A method for preparing the anti-CD24 antibody or antigen-binding fragment thereof according to any one of claims 1-10, which comprises a step for culturing the isolated host cell according to claim 13; optionally, the method further comprises a step for isolating the anti-CD24 antibody or antigen-binding fragment thereof.
